# EUROPEAN PATENT SPECIFICATION

(11) **EP 2 083 758 B1**
(45) Date of publication and mention of the grant of the patent: **01.11.2017**
(21) Application number: 07811404.8
(22) Date of filing: 17.08.2007
(51) Int. Cl.: A61F 2/38, G06G 7/48, G06G 7/58, A61F 2/30, A61B 90/00, A61F 2/36

(54) **SYSTEMS AND METHODS FOR DESIGNING, ANALYZING AND USING ORTHOPAEDIC DEVICES**
SYSTEME UND VERFAHREN FÜR DEN ENTWURF, ZUR ANALYSE UND ZUR VERWENDUNG VON ORTHOPÄDISCHEN VORRICHTUNGEN
SYSTÈMES ET PROCÉDÉS POUR LA CONCEPTION, L'ANALYSE ET L'UTILISATION DE DISPOSITIFS ORTHOPÉDIQUES

(30) Priority: 18.08.2006 US 506575; 06.11.2006 US 593294
(43) Date of publication of application: 05.08.2009
(73) Proprietor: Smith & Nephew, Inc., Memphis, TN 38116 (US)
(72) Inventor: MC COMBS, Daniel, L., Paw Paw, MI 49079 (US); MELTON, Mark, Carlsbad, CA 92008 (US); JAMES, Anthony, H., Bartlett, TN 38133 (US); BRUNEAU, Steve, Edgewater Park, NJ 08010 (US); RASSMANN, Thomas, W., Marlton, NJ 08053 (US); LANGLOIS, Jonathan, D., Wilton, NY 12831 (US)
(74) Representative: Smith & Nephew
(86) International application number: PCT/US2007/018260
(87) International publication number: WO 2008/021494

(56) References cited:
- WO-A2-02/076326
- US-A1- 2005 065 628
- US-A1- 2005 148 843
- US-B1- 6 205 411
- US-B1- 6 205 411

## Description

### RELATED FIELDS

The present invention relates to a method and a system for creating orthopaedic devices, such as biomedical implants. The closes prior art is document US 6205411 B1, which defines the preamble of the independent claims.

Some embodiments of the present invention may relate to systems and methods for designing, planning, and otherwise facilitating or enabling surgical procedures involving medical devices, such as biomedical implants, including the design, creation, and surgical implanting of an implant.

Some embodiments of the present invention may relate generally to biomechanical and other medical studies, such as methods executed by computer systems that provide information on, simulate, and/or model possible permutations of fracture types and medical device constructs.

Still other embodiments of the present invention may relate to systems or methods that employ combinations of at least some of the features of one or more of the other afore-mentioned embodiments.

### BACKGROUND OF THE INVENTION

Surgeons routinely implant biomedical implants, including but not limited to intramedullary nails or hip stems, into patients. For example, a patient might have fractured his or her leg after falling. Another patient might have conditions typically associated with old age, for example, a weakened hip. A surgeon might decide that the patient's condition requires treatment with one or more biomedical implants. There are companies that manufacture and sell biomedical implants in standard sizes and designs. Sometimes, these standard implants are appropriate for the patient's condition. The surgeon could simply purchase the standard implant to treat the patient's condition. But oftentimes, these standard biomedical implants are not appropriate for treatment of the patient's condition. For example, if a patient has a fractured leg, the fracture might be larger, wider, or otherwise differently shaped than is appropriate for a standard biomedical implant. The standard biomedical implant might not fit into the fracture, might not make contact at the required points, or might otherwise inhibit the treatment of the patient's condition.

When a standard biomedical implant is not appropriate for treatment, the surgeon can order a customized biomedical implant. Customized implants provide the flexibility of selecting an implant with the appropriate design to treat the patient's condition. Traditionally, biomedical implant manufacturers collaborate with a surgeon to design a customized implant. In this traditional method, all communication between the requesting surgeon and the biomedical implant manufacturer is either oral or written and delivered through the postal service. If the communication is oral, then the surgeon must schedule an appointment with the implant manufacturer, either by telephone or in person. Accordingly, communication can only occur when the manufacturer and surgeon are available at the same time. Waiting on concurrent availability takes a long time, and the delay might affect the patient's condition. Collaboration in these traditional methods of design also occurs in writing and is delivered through the postal service. For example, a surgeon might send X-rays and order forms through the mail, and the implant manufacturer will mail the design proposals back to the surgeon. Sending communication through the mail is time consuming, and the delay might affect the patient's condition. Accordingly, traditional systems and methods that depend on oral communication, or written communication delivered by the postal service, is undesirable.

Other traditional methods, such as the methods taught by Bradbury et al. in U.S. Patent No. 6,772,026, Funderund in U.S. Patent Application Publication No. 2005/0216305, or Baker in U.S. Patent Application Publication No. 2002/0072821 utilize a networked system that allows a surgeon's device to communicate electronically with a biomedical implant manufacturer's server and processor. In the systems taught by these references, the surgeon transmits to the manufacturer general patient data, such as gender, age, or name. The surgeon also transmits diagnostic data of the patient's condition, such as a digital X-ray image, a magnetic resonance image ("MRI"), or a computer tomography ("CT") image. The biomedical implant manufacturer receives this information on a server and or processor. In these traditional systems, the manufacturer's processor typically uses computer aided design ("CAD") systems to design a customized biomedical implant based on the received diagnostic data. In Bradbury et al., the design of the customized device is defined by edges of the patient's living tissue as shown in the patient's diagnostic data. So for example, if a patient has a fractured leg, the surgeon might transmit to the manufacturer a digital X-ray image that shows the two edges of the fractured leg. The system in Bradbury et al. receives the digital X-ray and uses a CAD system to design the customized device. The CAD system defines the edges of the customized device by the edges of the fractured leg as shown in the X-ray. These systems depend on the receipt of the diagnostic data, and the surgeon is not involved in the design of the customized device because the design is defined exclusively by the diagnostic data.

There are several drawbacks associated with traditional systems and methods such as those taught by Bradbury et al. and Funderund. Often the diagnostic data required by the systems is unreliable and inconsistent. The MRI or CT files can have errors that prohibit use by the manufacturer's CAD system. The submitting surgeon could make a mistake in the diagnostic data, or could submit the wrong type of diagnostic data. The transmission of diagnostic data through the network and servers of traditional systems also presents a problem. One acceptable framework for transmitting electronic diagnostic data is the Digital Imaging Communications in Medicine ("DICOM") standard, which is developed by the American College of Radiology ("ACR"). It is often difficult and costly, if not impossible, to convert diagnostic data to DICOM formats. The diagnostic data that is converted into DICOM formats is often unusable, and it is not reliable or verifiable for accuracy. Accordingly, it is not desirable to rely exclusively on diagnostic data to design the customized biomedical implant.

A second problem associated with traditional systems such as taught by Bradbury et al. and Funderund is the lack of collaboration between the surgeon and the device manufacturer in the design process. In traditional systems such as Funderund and Bradbury et al., the design is defined exclusively by the device manufacturer, and is based solely on the diagnostic data. In some cases, however, it might be desirable for the surgeon to contribute to the design independently of the diagnostic data. For example, the diagnostic data might indicate that a device should have a length of 3 mm, but the surgeon might want a length of 5 mm. Systems such as in Funderund and Bradbury et al. do not allow the surgeon to alter the design. Moreover, it would be difficult and costly to allow the surgeon to contribute to the design. These traditional systems use complicated CAD and CAM software of which the surgeon is most likely unaware. Even if the systems did allow the surgeon to alter the design, he or she could not effectively do so because of inexperience with the CAD and CAM software.

In other traditional systems such as in Baker, the design is defined exclusively by the user, and the device manufacturer cannot contribute to the design. In some situations, input might be required by the device manufacturer, for example if the proposed design is outside of acceptable ranges. Alternatively, the manufacturer might be aware of a standard device that could be substituted for the proposed design. It might be helpful for the manufacturer to be able to suggest the substitution. Systems such as in Baker do not allow the manufacturer to contribute to the design.

Yet another problem is that these traditional systems do not provide a method to trace design iterations. An originally submitted design might differ significantly from the final design. Oftentimes it is desirable to identify what changes were made to the design, when they were made, and who made them. These traditional systems and methods do not provide a way to track design changes.

Previously employed methodologies for custom implant design and use is also limited because an implant's design may depend on the associated surgical procedure, and vice versa. For example, in the context of arthroplasty (knee replacement) surgery, typically, standard implant parts are designed to interact with bone resections surgically made in locations and orientations appropriate for the implant parts. More specifically, these surgeries often involve resurfacing the knee joint by replacing a portion of the femur (thigh bone), tibia (shinbone), and/or patella (knee cap) with medical implants, which may be cemented or otherwise attached to the remaining portions of resected bone. Attachment of the implant, and therefore implant design, may in some cases thus depend on the altered anatomic structures created by resections and other surgical techniques.

Differences in both a patient's anatomy and details of an injury make every application of a surgical implant unique to some extent. In some cases, for example, the surgical site's condition (bone condition, surgical access, etc.) is not ideal or even suitable for resections in the standard locations and/or orientations. While various surgical techniques may be used to allow the use of standard implant parts in such non-ideal circumstances, e.g., using bone graft to help correct bone deficiencies, it would often be preferable to perform a "custom" surgery (e.g., cutting in non-standard locations and orientations) using a custom implant (e.g., one capable of attaching to the non-standard bone resection(s)). However, use of custom implants that requires non-standard cuts is hindered by several factors. For example, such efforts may be prevented by a lack of coordination in creating and using a custom implant in a "custom" surgical procedure. Such efforts may depend in part on coordinating information about the patient's physiology, the sensitivity of the surgical procedure to changes in technique and implant attributes, and the sensitivity of the medical device's performance to changes in implant attributes and surgical procedure. In addition, performing a non-standard surgical procedure (e.g., making non-standard cuts to accommodate a custom implant) may require additional knowledge or skill on the part of a performing surgeon or require different, or additional, medical equipment (guides, cutting blocks, etc). There is a need for additional systems and methods that provide for, or otherwise facilitate, designing, planning, and/or otherwise enabling non-standard surgical procedures involving custom medical devices.

Additionally, bone fractures, deformities and other conditions may be treated using a variety of medical devices. A surgeon selecting an appropriate medical device or devices for treatment of a fracture may take into account information about the fracture, the potential devices, the individual patient, and/or the potential stresses and motions to which the bone and device may be subject, among other things. In the case of plates used to treat bone fractures, in order to treat a fracture appropriately, surgeons and others desire to know or predict the proper plate/screw configuration to optimize the biomechanics of the particular fracture they are treating. They also frequently need to select between locking and traditional non-locked screws, which may perform in significantly different ways because of biomechanic differences. In short, selection of an appropriate medical device may be limited by a surgeon's limited ability to predict the performance of potential fracture and medical device constructs.

Various studies are underway to evaluate the plate/bone biomechanics of common fractures. However, there are many challenges in dealing with the many permutations of fracture, plate, and locked or unlocked screws. Biomechanics studies defining the biomechanics for a particular fracture/plate construct typically provide information only for the particular fracture/plate constructs and do not provide any ability to evaluate or optimize among multiple permutations of fracture type and constructs. Often surgeons do not have information about the specific biomechanics of the patients they are treating and simply rely on instinct and experience to select among alternative options.

The July 2004 thesis of Jonathan Kirk Nielson titled "Expedited Finite Element Analysis of Ankle External Fixation Stiffness," which is incorporated herein by this reference, discloses software for measuring Ilizarov distal tibia frame axial stiffness that utilizes finite element analysis software. The software allows a user to specify different wire and pin configurations as input and delivers axial movement of the tibia as output. However, the approach does not include the actual bone or fracture(s) in the bone in the characterization.

There is a need for enhanced simulation capabilities and, in the context of plate devices, there is a need for simulation of performance of alternative plate/screw configurations that takes into account actual bone, plate type, and/or screw configuration characteristics. Such simulation may allow selection of among alternative configurations to optimize biomechanics and provide more rapid healing, fewer non-unions and failures. There is a further need for a computer system that offers adequate simulation that allows a surgeon to input information about the fracture type and characteristics, as well as potential plate and screw combinations, and choose the appropriate plate and locked or unlocked screw configuration to optimize the biomechanics for the fracture and other bone characteristics of the patient. Similar needs also exist for other types of devices, including, but not limited to, screws, external fixators and other devices used in orthopaedic or trauma applications.

### SUMMARY OF THE INVENTION

The invention is defined in claims 1 and 5. Embodiments of the invention provide for systems and methods of creating and obtaining biomedical implants. Certain embodiments may include a computer, facsimile machine, handheld computing device, or a telephone system, if desired. Other embodiments may have an automated platform for an implant manufacturer, which can also be embodied as a computer, facsimile machine, handheld computing device, or a telephone system, if desired. Embodiments may include a server to allow the surgeon's device and the automated platform to transmit information over a network. In some embodiments, the network may be the Internet while in others, the network might be an Intranet. Certain embodiments may further include a manufacturing facility. The manufacturing facility may receive instructions to manufacture the implant, and may then send the implant to the surgeon.

In some embodiments, the surgeon may be provided with tools, or may have access to tools, to enable the surgeon to practice certain methods of the invention. The tools may be embodied as software loaded on a computer, if the surgeon's device is embodied as a computer. In an alternative embodiment, the tools may be provided on the automated platform or on a server, and can be accessible by the surgeon's device. If desired, the tools may be other known embodiments such as menu selections on a telephone system. One tool may allow a surgeon to transmit a request for a biomedical implant. Tools of other embodiments may enable the surgeon to edit templates that represent biomedical implants. Embodiments may further enable the surgeon to upload or input information into the surgeon's device, such as diagnostic data and patient information, if desired. Certain embodiments may enable the surgeon to either send or receive information.

Some embodiments may include a server to receive and transmit information between the surgeon's device and the automated platform, if provided in the embodiment. If desired, the server may transmit information over the Internet. The server and the automated platform may be housed in the same location, or may be housed in different locations. In some embodiments, the server and the automated platform may be members of the same element, or the server and the automated platform may be separate elements.

Some embodiments of the invention may include an automated platform that can be accessed by a biomedical implant manufacturer. The automated platform may be embodied as a computer, if desired, and may also be embodied as a facsimile machine, handheld computing device, or a telephone system. In some embodiments there are tools associated with the automated platform, and the tools can include computer aided design ("CAD") systems. The CAD systems may be enabled to translate edits received from the surgeon's device into a design for an implant. In some embodiments, memory can be provided. The automated platform may access the memory, but the automated platform and the memory need not be members of the same element. The memory can include multiple types of information, including a collection of templates, ranges of acceptable design parameters for implants, or designs of standard implants.

In some embodiments, there is provided a manufacturing facility. The manufacturing facility may receive manufacturing instructions from the automated platform. The manufacturing facility and the automated platform may communicate through the server, or by other means including the postal service or telephone system. The manufacturing facility may have known manufacturing tools, and can manufacture the implant. If desired, the manufacturing facility can send the implant to the surgeon.

Some embodiments of the invention provide systems and methods for creating and obtaining devices, including biomedical implants.

Some embodiments of the invention provide systems and methods that quickly and efficiently create implants.

Some embodiments of the invention provide systems and methods that create implants without the need for diagnostic data.

Some embodiments of the invention provide systems and methods that allow a surgeon and an implant manufacturer to collaborate on a proposed implant design.

Some embodiments of the invention provide systems and methods that allow a surgeon who is unskilled in CAD systems to contribute to a proposed implant design.

Some embodiments of the invention provide systems and methods that record pertinent information about design iterations.

The above-mentioned, or other embodiments of the invention may provide systems and methods for planning surgical procedures involving medical implants. Certain embodiments involve the selection, design and/or creation of custom medical implants and/or the selection, modification, and/or design of custom surgical procedures related to those implants. Certain embodiments may allow implants to be selected, designed, created, or otherwise customized and then placed in patients using surgical techniques that are different from the techniques used with standard medical implants or that otherwise alter the structure of the patient's anatomy to have attributes that allow the implant to be attached.

Some embodiments of the invention provide systems and methods that provide greater flexibility in implant use by allowing a surgeon and/or others to revise, create, or otherwise select surgical techniques for custom implants and, ultimately, provide better treatment in a greater variety of medical circumstances.

Some embodiments of the invention may relate to computer aided surgery (CAS) systems and methods. Some embodiments may provide to a CAS system information regarding a surgery and/or an implant, either or both of which may have custom components. For example, the CAS system may use 3D model geometry to display an image representative of an actual custom implant being used. Alternatively, the preplanning stage of a CAS surgery and the design of a custom implant and associated custom surgical steps may be combined in a combined planning process that allows a surgeon to design and plan the CAS surgery using a single integrated tool. In either case, the CAS system may, in some embodiments, then be used to facilitate the surgical procedure.

Some embodiments of the invention relate to systems and methods that provide computer-controlled modifications to implant surgical procedure to accommodate non-standard implants and/or non-standard surgical procedures in conjunction with associated CAS systems and processes.

The above-mentioned, or other embodiments, may provide methods of selecting a bone, plate, and configuration of fixation and compression screws for the bone plate to be installed on a bone of a patient. Such methods may involve inputting into a computer, having a processor, memory and input / output functionality, information about one or more of (1) the bone; (2) the patient; (3) static and dynamic physical forces to which the bone will be subjected; (4) a candidate bone plate; and (5) fixation and compression screws adapted to be potentially used with the plate. Such methods may also involve simulating in the computer (1) the bone plate secured to the bone using a first configuration of fixation and compression screws, and (2) performance of the structure comprising the bone plate, bone and the first configuration of the fixation and compression screws. Such methods may also involve simulating in the computer (1) the bone plate secured to the bone using a second configuration of fixation and compression screws, and (2) performance of the structure comprising the bone plate, bone and the second configuration of the fixation and compression screws. Such methods may also involve selecting a selected configuration of fixation and compression screws based on these simulations.

These methods may involve using finite element analysis for simulating performance of the structure comprising the bone plate, bone, and first configuration of the fixation and compression screws and/or simulating performance of the structure comprising the bone plate, bone, and second configuration of the fixation and compression screws. Other embodiments may involve providing the selected configuration to a user over the world wide web. Yet other embodiments may involve each screw configuration having, for each aperture in the locking plate, either a non-locking screw, a locking screw, or no screw. In yet other embodiments, the method may further involve providing an interface for a remote user to input constraints for the simulation over the Internet, and/or providing the selected configuration to the remote user.

Some other embodiments of the invention may involve methods for using a computer to evaluate one or more options for associating at least one surgical device with a bone. A standard computer may be used and will typically include processing functionality, memory functionality, input functionality and output functionality. The computer may accept a plurality of information elements as input. An information element may be any piece of information or data that is stored in any suitable data format, e.g., in a database record, a file, a message, etc. The method may involve (a) inputting at least one first information element into the computer concerning the bone, (b) inputting at least one second information element into the computer concerning the surgical device, (c) inputting at least one third information element into the computer concerning a scenario for associating the surgical device with the bone, (d) using the computer, analyzing the first, second and third information elements, (e) using the computer, outputting at least one fourth information element predicting at least one performance characteristic of the scenario, and (f) using the fourth information element to evaluate whether to follow the scenario to associate the surgical device with the bone or to otherwise evaluate and/or record the biomechanical construct. The first information element may further concern a fracture in the bone, information about the fracture that is rendered on a computer image of a bone, the side of the bone the fracture is on, the type of fracture, and/or the bone condition, among other things. The second information element may further concern the type of the device, the position of the device, information about the selection of at least one screw for use in a hole in the device, and/or information about the characteristics of a screw for use in a hole in the device. Inputting the second information element may further comprise displaying a plurality of holes on a graphical image of the device affixed to the bone and selecting a hole on the image of the device for a screw. The method may further involve optimizing screw configuration by running iterations to minimize one or more of stress and motion.

In some embodiments, one or more of the information elements may be inputted using interactive screens or menus displayed by the computing device. The data for input may be gathered in any suitable or desirable manner, including, but not limited to, digital or non-digital x-ray technology, fluoroscopy technology, computer assisted surgical navigation technology, measuring devices, databases, kinetic evaluation, or other types of evaluation. In some embodiments, the computer may communicate with the device or systems used to gather the data, eliminating the need for the user to input the data. Rather, the systems may, in some embodiments, automatically enter or input the data.

Some embodiments may involve a variety of types and configurations of surgical devices providing conventional, non-conventional, and/or a combination of conventional and non-conventional fixation means including plates, screws, pegs, nails, external fixators, bioresorbably fixation elements, bone glue, etc. In certain embodiments, a surgeon may use some embodiments to evaluate which size hip or knee implant optimizes the biomechanics. This is frequently a combination of the amount of bone to take out versus the size of implant to use. For example, in some situations, it may be prudent to remove more bone to get a larger implant in place, yielding a better transfer of load to the bone. In other cases, it may be better to preserve bone and use a smaller implant. This could apply to hip femoral components, knee femoral and tibia components, acetabulum components, etc.

Some embodiments may provide systems and methods for designing, analyzing and using orthopaedic devices. Systems and methods in accordance with at least some of these embodiments may involve obtaining data concerning a patient's anatomy, such as X-rays, digital X-rays, magnetic resonance images ("MRI"), computer tomography ("CT") images, or other types of data, including data not necessarily associated with an image, such as data concerning mechanical axes or relative positions of landmarks of the anatomy of interest. In some embodiments, this data may be used to manufacture customized orthopaedic implants, instruments, or other devices. For instance, in some embodiments, the data may be used to manufacture a custom cutting block for facilitating the at least partial resection of the anatomy of interest to receive a desired implant. In another example, the data may be used to manufacture both a custom cutting block and a custom implant for implantation onto the resected surfaces at least partially created using the custom cutting block. In some embodiments, the manufacture of the customized orthopaedic devices may be accomplished through a semi to fully automated process, although, in other embodiments, manufacturing processes involving varying degrees of human involvement may be used as well or in the alternative. In some embodiments, computer assisted design programs or other computer functionality may be used to develop specifications, parameters, manufacturing instructions, or other information used to manufacture the customized orthopaedic device.

In some embodiments, the data may be transmitted to the manufacturer electronically, such as over a network connection, such as an intranet or Internet, and may involve two-way exchanges of data, just a single direction data transmission, or other types of data exchanges.

In some embodiments, the customized orthopaedic device(s) may be packaged along with other customized or non-customized orthopaedic devices or other items and provided to a surgeon for use in an orthopaedic procedure on the patient's anatomy.

There is provided a method of creating an orthopaedic device, comprising receiving information about a surgical site of a patient, using the information about the surgical site to determine a surgical procedure step for creating an anatomic structure attribute, using information about the anatomic structure attribute to determine a custom attribute for an orthopaedic device, and manufacturing the orthopaedic device to include the custom attribute, wherein using the information about the surgical site further comprises estimating a level of difficulty of the surgical procedure step.

According to some embodiments, the method further comprises estimating a minimum level of skill required to perform the surgical procedure step.

According to some embodiments, manufacturing the orthopaedic device may comprise manufacturing a cutting block for guiding the resection of a portion of a bony anatomy associated with the surgical site.

According to some embodiments, manufacturing the orthopaedic device may comprise manufacturing a knee implant for replacing a portion of a bony anatomy associated with the surgical site.

There is provided a system for creating an orthopaedic device, comprising a platform for (i) receiving information about a surgical site of a patient, (ii) determining a surgical procedure step for creating an anatomic structure attribute using the information about the surgical site, (iii) determining a custom attribute for an orthopaedic device using the anatomic structure attribute, and (iv) estimating a level of difficulty of the surgical procedure step using the information about the surgical site, and at least one manufacturing device for manufacturing the orthopaedic device to include the custom attribute.

According to some embodiments, the platform estimates a minimum level of skill required to perform the surgical procedure step.

According to some embodiments, the orthopaedic device may comprise a cutting block for guiding the resection of a portion of a bony anatomy associated with the surgical site.

According to some embodiments, the orthopaedic device may comprise a knee implant for replacing a portion of a bony anatomy associated with the surgical site.

Also described is a method for biomedical implant design and procurement, comprising in an automated platform for design of biomedical implants, receiving a request for a biomedical implant from a surgeon's device adapted to communicate with the automated platform, accessing memory of the automated platform to obtain a template corresponding to the request, automatically transmitting the template to the surgeon's device, providing a tool for editing the template using the surgeon's device, receiving at least a first edit from the surgeon's device, automatically generating at least a first response to the at least a first edit, automatically transmitting the at least a first response to the surgeon's device, receiving approval from the surgeon's device, and automatically creating a final design for the biomedical implant.

The first response may comprise at least automatically translating the at least a first edit into an at least a first design for a biomedical implant.

The first response may comprise at least accessing memory of the automated platform to obtain an acceptable range of design parameters and automatically generating a notice if the at least a first edit falls outside of the acceptable range.

The first response may comprise at least accessing memory of the automated platform to obtain a design of a standard biomedical implant and automatically generating a notice if the final design is substantially similar to the design of a standard biomedical implant.

The tool may be provided on at least one of the surgeon's device or the automated platform.

The tool may comprise at least one of a slideable bar associated with an edge of the template, or a form to input design parameters.

The template may be for an intramedullary nail and the tool may be adapted to enable editing of at least one of the length, outside diameter, bow radius, hole size, hole location, hole angle, the number of holes, slot size, slot location, material, surface finish, or surface coating.

The template may be for a hip stem and the tool may be adapted to enable editing of at least one of the profile, stem size, length, head offset, neck height, neck angle, medial and lateral fill, posterior and anterior fill, shoulder height, beam thickness, material, surface finish, or surface coating.

According to some examples, the method further comprises providing a tool for comparison of the final design to diagnostic data.

According to some examples, the method further comprises generating machine instructions and sending the machine instructions to manufacturing facility.

According to some examples, method further comprises manufacturing the biomedical implant and sending the biomedical implant to the surgeon.

Also described is a method for biomedical implant design and procurement, comprising in an automated platform for design of biomedical implants, receiving a request for a biomedical implant from a surgeon's device which device is adapted to communicate with the automated platform, accessing memory of the automated platform to obtain a template corresponding to the request, automatically transmitting the template to the surgeon's device, providing a tool for editing the template using the surgeon's device, receiving at least a first edit from the surgeon's device, accessing memory of the automated platform to obtain an acceptable range of design parameters and automatically generating a notice if the at least a first edit falls outside of the acceptable range, automatically transmitting the notice to the surgeon's device, receiving approval from the surgeon's device, and automatically creating a final design for the biomedical implant.

According to some examples, the tool may comprise at least one of a slideable bar associated with an edge of the template or a form to input design parameters.

According to some examples, the method further comprises generating machine instructions and sending the machine instructions to manufacturing facility.

According to some examples, the method further comprises manufacturing the biomedical implant and sending the biomedical implant to the surgeon.

According to some examples, the method further comprises repeating the steps on at least a second edit.

Also described is a method for biomedical implant design and procurement, comprising in an automated platform for design of biomedical implants, receiving a request for a biomedical implant from a surgeon's device which device is adapted to communicate with the automated platform, accessing memory of the automated platform to obtain a template corresponding to the request, automatically transmitting the template to the surgeon's device, providing a tool for editing the template using the surgeon's device, receiving at least a first edit from the surgeon's device, accessing memory of the automated platform to obtain designs of standard biomedical implants, automatically generating a notice if the at least a first edit is substantially similar to the design of a standard biomedical implant, automatically transmitting the notice to the surgeon's device, receiving approval from the surgeon's device, and automatically creating a final design for the biomedical implant.

According to some examples, the tool for editing may comprise at least one of a slideable bar associated with an edge of the template or a form to input design parameters.

According to some examples, the method further comprises automatically generating machine instructions and sending the machine instructions to manufacturing facility.

According to some examples, the method further comprises manufacturing the biomedical implant and sending the biomedical implant to the surgeon.

Also described is a method for biomedical implant design and procurement, comprising receiving a request for a biomedical implant from a surgeon's device which device is communicatively linked with an automated platform for design of biomedical implants, accessing memory to obtain a template corresponding to the request, providing a tool for editing the template using the surgeon's device where the tool comprises at least one of a slideable bar associated with an edge of the template or a form to input design parameters, receiving at least a first edit from the surgeon's device, automatically generating at least a first response to the at least a first edit, automatically transmitting the at least a first response to the surgeon's device, receiving approval from the surgeon's device, and automatically creating a final design for a biomedical implant.

According to some examples, the first response may comprise at least automatically translating the at least a first edit into an at least a first design for the biomedical implant.

According to some examples, the first response may comprise at least accessing memory to obtain an acceptable range of design parameters and automatically generating a notice if the at least a first edit falls outside of the acceptable range.

According to some examples, the method further comprises providing particular design parameters in the second form in response to the design parameters selected in the first form.

According to some examples, the method further comprises providing a tool for comparison of the final design to diagnostic data.

According to some examples, the method further comprises generating machine instructions and sending the machine instructions to manufacturing facility.

According to some examples, the method further comprises manufacturing the implant and sending the implant to the surgeon.

According to some examples, the method further comprises repeating steps on at least a second edit.

According to some examples, the template may be for an intramedullary nail and the tool may be adapted to enable editing of at least one of the length, outside diameter, bow radius, hole size, hole location, hole angle, the number of holes, slot size, slot location, material, surface finish, or surface coating.

According to some examples, the template may be for a hip stem and the tool may be adapted to enable editing of at least one of the profile, stem size, length, head offset, neck height, neck angle, medial and lateral fill, posterior and anterior fill, shoulder height, beam thickness, material, surface finish, or surface coating.

Also described is a method for biomedical implant design and procurement, comprising in an automated platform for design of biomedical implants, receiving a request for a biomedical implant comprising at least diagnostic data from a surgeon's device which device is adapted to communicate with the automated platform, providing a tool for selecting points associated with the diagnostic data using the surgeon's device, receiving a selection from the surgeon's device, automatically translating the selection into an at least a first design for a biomedical implant, automatically transmitting the at least a first design to the surgeon's device, providing a tool for editing the at least a first design using the surgeon's device, receiving approval from the surgeon's device, automatically creating a final design for the biomedical implant and machine instructions and sending the machine instructions to a manufacturing facility, and manufacturing the biomedical implant and sending the biomedical implant to the surgeon.

Also described is a method for biomedical implant design and procurement, comprising in an automated platform for design of biomedical implants, receiving a request for a biomedical implant from a surgeon's device which device is adapted to communicate with the automated platform, accessing memory of the automated platform to obtain a template corresponding to the request, automatically transmitting the template to the surgeon's device, providing a tool for editing the template using the surgeon's device, receiving at least a first edit from the surgeon's device, automatically generating at least a first response to the at least a first edit, automatically transmitting the at least a first response to the surgeon's device, receiving approval from the surgeon's device, automatically creating machine instructions and sending the machine instructions to a manufacturing facility, and manufacturing the biomedical implant and sending the biomedical implant to the surgeon.

According to some examples, the tool for editing may comprise at least one of a slideable bar associated with an edge of the template or a form to input design parameters.

According to some examples, the first response may comprise at least translating the at least a first edit into an at least a first design for a biomedical implant.

According to some examples, the method further comprises repeating steps on at least a second edit.

Also described is a system for biomedical implant design and procurement, comprising a template for a biomedical implant, a surgeon's device adapted to allow editing of the template using a tool, an automated platform enabled to receive at least a first edit and to automatically generate at least a first response, and a server to communicatively link at least the surgeon's device and the automated platform.

According to some examples, the tool may comprise at least one of a slideable bar associated with an edge of the template, or a form to input design parameters.

According to some examples, the tool may be provided on at least the surgeon's device or the automated platform and is accessible by the surgeon's device.

According to some examples, the first response may comprise at least automatically translating the at least a first edit into an at least a first design for a biomedical implant

According to some examples, the first response may comprise at least accessing memory of the automated platform to obtain an acceptable range of design parameters and generating a notice if the at least a first edit falls outside of the acceptable range.

According to some examples, the first response may comprise at least accessing memory of the automated platform to obtain a design of a standard biomedical implant and automatically generating a notice if the at least a first edit is substantially similar to the design of a standard biomedical implant.

According to some examples, the first response may further comprise providing a tool adapted to enable comparison of the at least a first design to diagnostic data.

According to some examples, the automated platform may be enabled to generate machine instructions, and the system may further comprise a manufacturing facility to manufacture the biomedical implant and send the biomedical implant to the surgeon.

Also described is a system for biomedical implant design and procurement, comprising a template for a biomedical implant, a surgeon's device adapted to allow editing of the template using a tool, the tool comprising at least one of a slideable bar associated with an edge of the template or a menu for inputting design parameters, an automated platform enabled to receive at least a first edit and to automatically translate the at least a first edit into an at least a first design for a biomedical implant, and a server to communicatively link at least the surgeon's device and the automated platform.

According to some examples, the automated platform may be further enabled to access memory to obtain an acceptable range of design parameters and to automatically generate a notice if the at least a first edit falls outside of the acceptable range.

According to some examples, the automated platform may be further enabled to access memory to obtain a design of a standard biomedical implant and to automatically generate a notice if the at least a first edit is substantially similar to the design of a standard biomedical implant.

According to some examples, the automated platform may be further enabled to generate machine instructions, and the system may further comprise a manufacturing facility to manufacture the implant and send the implant to the surgeon.

Also described is a system for biomedical implant design and procurement, comprising a template for a biomedical implant, a surgeon's device adapted to allow editing of the template using a tool, an automated platform enabled to receive at least a first edit and to automatically generate a notice if the at least a first edit is substantially similar to the design of a standard biomedical implant, a server to communicatively link at least the surgeon's device and the automated platform.

Also described is a system for biomedical implant design and procurement, comprising a template for a biomedical implant, a surgeon's device adapted to allow editing of the template using a tool, an automated platform enabled receive at least a first edit and to automatically generate a notice if the at least a first edit falls outside of an acceptable range of design parameters, and a server to communicatively link at least the surgeon's device and the automated platform.

Also described is a method of planning and executing a medical implant surgical procedure comprising receiving information about a surgical site of a patient, using the information about the surgical site to determine a surgical procedure step for creating an anatomic structure attribute that allows attachment of a medical implant, using information about the anatomic structure attribute to determine a custom attribute of the medical implant for attaching the medical implant to the anatomic structure attribute, creating the medical implant comprising the custom attribute, and implanting the medical implant at the surgical site by following the surgical procedure step and attaching the medical implant custom attribute to the anatomic structure attribute.

According to some examples, using information about the anatomic structure attribute to determine a custom attribute of the medical implant may further comprise estimations of implant performance after surgeries.

According to some examples, using the information about the surgical site to determine the surgical procedure step may further comprise estimations of the difficulty of the surgical procedure step.

According to some examples, using the information about the surgical site to determine the surgical procedure step may further comprise estimations of a minimum level of skill required to perform the surgical procedure step.

Also described is a system for planning a medical implant surgical procedure comprising a user interface that allows a user to enter information about a surgical site, and a display component that provides an image representative of a custom attribute of a medical implant for allowing attachment at the surgical site and an image representative of an altered anatomic structure attribute for attachment of the custom attribute of the medical implant, wherein the system allows the user to make adjustments that result in changes to the image representative of the custom attribute of the medical device and the image representative of the attribute of the altered anatomic structure.

According to some examples, the image representative of the custom attribute of the medical device and the image representative of the attribute of the altered anatomic structure may be displayed overlapping one another.

According to some examples, the image representative of the custom attribute of the medical device and the image representative of the attribute of the altered anatomic structure may be displayed adjacent to one another.

According to some examples, the system may allow the user to make adjustment through the user interface, and the user interface may allow the user to graphically make adjustments.

According to some examples, user adjustments may be constrained by estimations of implant performance.

Also described is a method of planning and executing a medical implant surgical procedure comprising receiving input relating to an attribute of an anatomic structure for attachment of an implant, using the input to determine a custom attribute of the implant for attachment to the anatomic structure attribute, providing to a CAS system information about the custom implant attribute and information about the anatomic structure attribute, and providing during surgery from the CAS system an image representative of the custom implant attribute and an image representative of the anatomic structure attribute.

According to some examples, the image representative of the custom implant attribute may overlap the image representative of the anatomic structure attribute.

According to some examples, the image representative of the custom implant attribute may be adjacent to the image representative of the anatomic structure attribute.

According to some examples, the CAS system may further provide information regarding surgical steps associated with creating a surgical site anatomy according to the image representative of the anatomic structure attribute.

Also described is a method of selecting a configuration of fixation and compression screws for a bone plate to be installed on a bone of a patient, comprising inputting into a computer, having a processor, memory and input / output functionality, information about one or more of the bone, the patient, physical forces to which the bone will be subjected, a candidate bone plate, fixation and compression screws adapted to be potentially used with the plate, simulating in the computer the bone plate secured to the bone using a first configuration of fixation and compression screws, and performance of the structure comprising the bone plate, bone and the first configuration of the fixation and compression screws, simulating in the computer the bone plate secured to the bone using a second configuration of fixation and compression screws, and performance of the structure comprising the bone plate, bone and the second configuration of the fixation and compression screws, and selecting a selected configuration of fixation and compression screws based on said simulations.

According to some examples, finite element analysis may be used for simulating performance of the structure comprising the bone plate, bone, and first configuration of the fixation and compression screws, and simulating performance of the structure comprising the bone plate, bone, and second configuration of the fixation and compression screws.

According to some examples, the method further comprises providing the selected configuration to a user over the world wide web.

According to some examples, each screw configuration may have, for each aperture in the locking plate, either a non-locking screw, a locking screw, or no screw.

According to some example, the method further comprises providing an interface for a remote user to input constraints for the simulation over the Internet, and providing the selected configuration to the remote user.

According to some examples, the physical forces to which the bone will be subjected may comprise static and dynamic forces.

According to some examples, the physical forces to which the bone will be subjected may comprise static forces only.

According to some examples, the method further comprises selecting the selected configuration based at least in part on which configuration produces an optimum force mode.

According to some examples, the method further comprises selecting the selected configuration based at least in part on which configuration produces the best stress distribution for healing the bone.

Also described is a method for using a computer to evaluate at least one option for associating at least one surgical device with at least one bone, the computer comprising processing functionality, memory functionality, input functionality and output functionality, the method comprising inputting at least one first information element into the computer concerning the bone, inputting at least one second information element into the computer concerning the device, inputting at least one third information element into the computer concerning a scenario for associating the surgical device with the bone, using the computer, analyzing the first, second and third information elements, using the computer, outputting at least one fourth information element predicting at least one performance characteristic of the scenario, and using the fourth information element to evaluate the scenario.

According to some examples, the first information element may further concern a fracture in the bone.

According to some examples, the first information element may further concern information about the fracture that is drawn on a computer image of a bone.

According to some embodiments, the first information element may further concern the side of the bone the fracture is on.

According to some example, the first information element may further concern the type of fracture.

According to some examples, the first information element may further concern the bone condition.

According to some examples, the second information element may further concern the type of the device.

According to some examples, the second information element may further concern the position of the device.

According to some examples, the second information element may further concern information about the selection of at least one screw for use in a hole in the device.

According to some examples, inputting the second information element may comprise displaying a plurality of holes on a graphical image of the device affixed to the bone and selecting a hole on the image of the device for a screw.

According to some examples, the second information element may further concern information about the characteristics of a screw for use in a hole in the device.

According to some examples, the third information element may further concern a stress distribution on the bone.

According to some examples, the third information element may further concern a stability analysis on the bone.

According to some examples, the method further comprises optimizing screw configuration by running iterations to minimize one or more of stress.

According to some examples, the method further comprises optimizing screw configuration by running iterations to minimize one or more of motion.

Also described is a method of selecting a configuration of fixation and compression screws for a bone plate to be installed on a bone of a patient, comprising inputting into a computer, having a processor, memory and input / output functionality, information about one or more of (1) the bone, (2) the patient, (3) physical forces to which the bone will be subjected, (4) a candidate bone plate, and (5) fixation and compression screws adapted to be potentially used with the plate.

### GENERAL DESCRIPTION OF THE DRAWINGS

Figure 1 illustrates a system that can be used to practice an embodiment of a method of this invention.
Figure 2 illustrates an embodiment of the method in Figure 1.
Figure 3 is another illustration of the method in Figure 2.
Figure 4 illustrates a portion of a method of certain embodiments of the invention.
Figure 5 illustrates another portion of a method of certain embodiments of the invention.
Figure 6 illustrates a tool to send a request for a biomedical implant.
Figure 7 illustrates a tool for editing a hip stem template, where the tool comprises at least one slideable bar.
Figure 8 illustrates a tool for editing a hip stem template, where the tool comprises at least one form to input design parameters.
Figure 9 illustrates a tool for editing an intramedullary nail, where the tool comprises at least one form to input design parameters.
Figure 10 illustrates another example of the tool in Figure 9.
Figure 11 illustrates a tool to compare diagnostic data with a design for a biomedical implant.
Figure 12 illustrates an exemplary design and CAS system.
Figure 13 is a an exemplary implant illustrating non-standard geometry.
Figure 14 illustrates a computer interface for inputting and displaying information about a case.
Figure 15 illustrates a computer interface for inputting and displaying information about a bone and/or fracture.
Figure 16 illustrates a computer interface for inputting and displaying information about an device, bone, and/or fracture.
Figure 17 illustrates a computer interface for selecting analysis of stress distribution, stability, and/or optimization.
Figure 18 illustrates a computer interface for displaying a report.

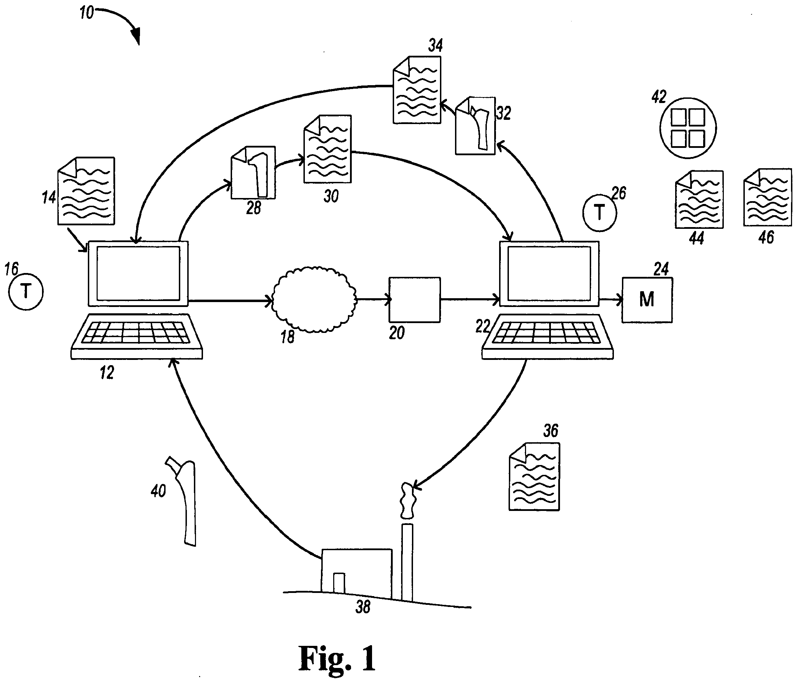

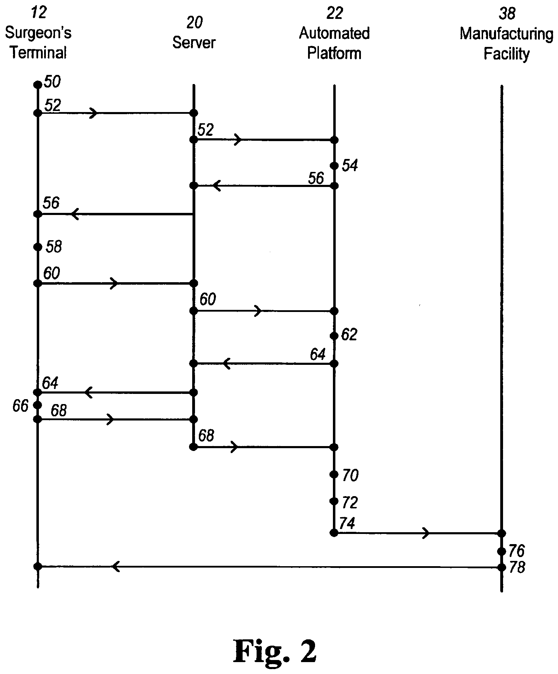

### DETAILED DESCRIPTION OF THE DRAWINGS Figures 1 Through 11

Some embodiments of the invention provide methods for creating and obtaining devices, including biomedical implants. Referring now to Figures 1-3, systems 10 can be provided with a surgeon's device 12. In Figure 1, the surgeon's device 12 is embodied as a computer, but other embodiments can include a facsimile machine, a telephone, a handheld computing device, or a pager. The surgeon's device 12 can access a tool 16 to input information 14. The tool 16 can be provided on the surgeon's device 12, or can be provided on the automatic platform 22 or the server 20 and can be accessible by the surgeon's device 12. Information 14 can include identifying information for the surgeon and/or the patient, including names, case numbers, and insurance information, if desired. In certain examples, information 14 can include diagnostic data, such as a digital X-ray image, a magnetic resonance image ("MRI"), or a computer tomography ("CT") image.

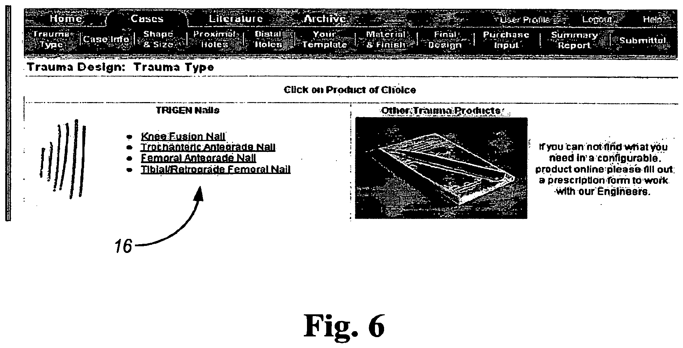

The surgeon's device 12 can access other tools 16 to practice other methods. The tools 16 can be provided on the surgeon's device 12, or can be provided on the automatic platform 22 or the server 20 and can be accessible by the surgeon's device 12. One tool 16 can enable the surgeon to send a request 28 for a template 32 that represents a biomedical implant, or for an accessory to a biomedical implant. Figure 6 illustrates a tool 16 to send a request 28 for template 32 of an intramedullary nail. The tool 16 provides a choice of four types of nails: a knee fusion nail, a trochanteric antegrade nail, a femoral antegrade nail, and a tibial/retrograde femoral nail. Other types of implants can also be included. For example, implants for different types of hip stems, or for cosmetic implants such as breast implants can be provided. Accessories or customized instruments to implant the implants can also be provided. For example, sometimes when a surgeon installs a custom-designed implant, the surgeon requires special tools. Accordingly, systems 10 can provide accessories to help the surgeon install the biomedical implant. For example, there can be provided drills, drill guides, broaches, nail guide drops, nail guide handles, proximal entry reamers, reamer drills, hexdrivers, drill sleeves, guide bolts, targeters, or other accessories known in the art. Further, embodiments of the invention can provide templates for trays in which to house accessories and/or biomedical implants. Accordingly, the surgeon's request 28 can include multiple types of implants, or accessories for implants, or trays for implants.

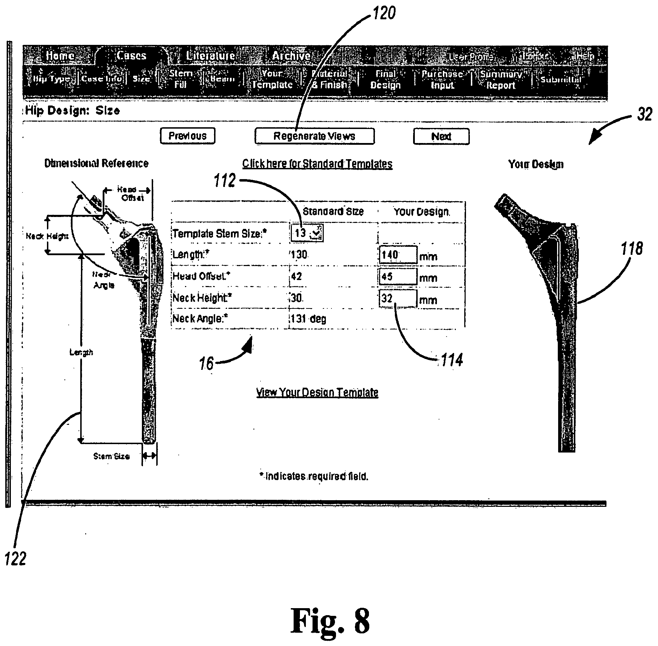

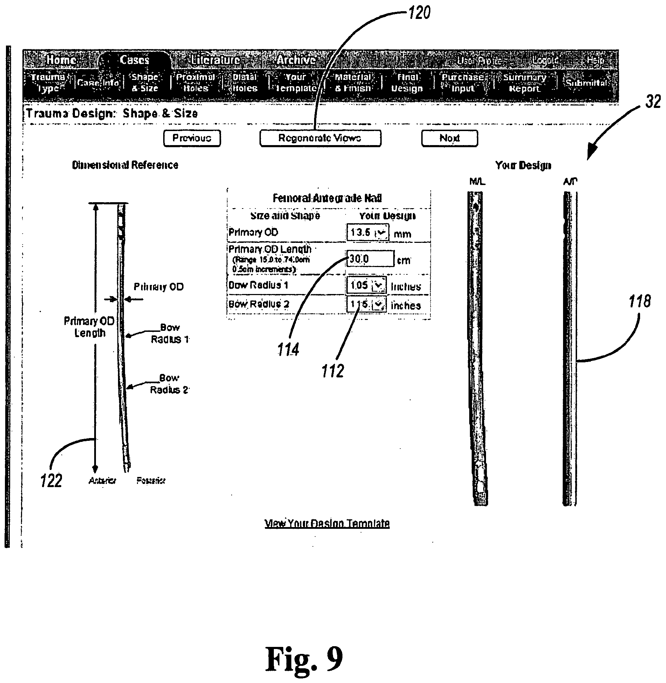

Certain arrangements also provide tools 16 for editing at least one design parameter of a template 32. The tools 16 can be provided on the surgeon's device 12, or can be provided on the automatic platform 22 or the server 20 and can be accessible by the surgeon's device 12. There can be many design parameters associated with various templates 32. For example, if the implant is for an intramedullary nail, as in Figure 9, examples of design parameters can include length, outside diameter, bow radius, hole size, hole location, hole angle, the number of holes, slot size, slot location, material, surface finish, or surface coating. If the implant is for a hip stem, as in Figure 8, examples of design parameters can include the profile, stem size, length, head offset, neck height, neck angle, medial and lateral fill, posterior and anterior fill, shoulder height, beam thickness, material, surface finish, or surface coating.

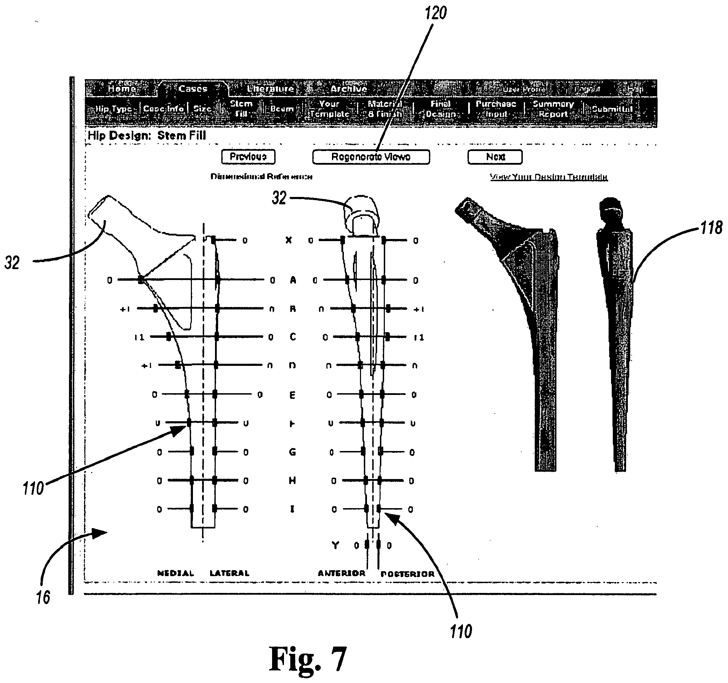

One example of tool 16 for editing is illustrated in Figure 7 and can include at least one slideable bar 110 associated with an edge of the template 32. The surgeon can click and drag the slideable bar 110 to adjust design parameters of the template 32. As illustrated in Figure 7, the slideable bars 110 can adjust the width and depth of the template 32_{.} In other examples, the slideable bars 110 can adjust the height or other design parameters of the template 32. The invention is not limited to a slideable bar 110, but can also include slideable points, or any other shaped sliding element.

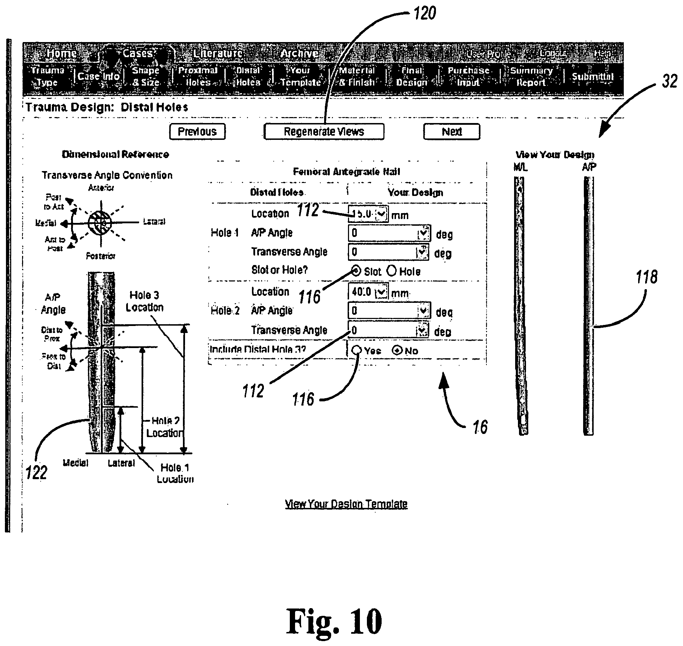

In the example illustrated in Figures 8-10, the tool 16 can include a form to input at least one design parameter. There can be at least three types of forms: a menu 112, a blank field 114, or a selector 116. If the form is embodied as a menu 112, the surgeon can click on the down arrow to obtain a list of design parameters, and can click on the desired design parameter of his/her choice. In Figure 9, there is provided three menus 112, two of which are consecutive. In certain examples, menus 112 can be interrelated such that their design parameters depend on one another. In these examples, the design parameters presented for selection in the second menu 112 depend on the design parameters that the surgeon selected in the first menu 112. For example, if in the first menu 112 the surgeon selected an outside diameter of 10 mm, then the choices for an inside diameter provided in the second menu 112 would be less than 10 mm. If the form is embodied as a blank field 114, the surgeon can type in the desired design parameter into the blank field 114. Still another embodiment of a tool 16 is a selector 116 for the surgeon to select design parameters. In Figure 10, there are two selectors 116 and each selector 116 only provides two choices. In other examples, there can be multiple selectors 116 and each selector 116 can provide multiple choices. Accordingly, the tool 16 for editing can include at least one of a slideable bar 110, a menu 112, a blank field 114, a selector 116, or a combination of any of these.

The examples in Figures 8-10 illustrate a dimensional reference 122, which can serve as a guide while the surgeon is editing the template 32. For example, the dimensional reference 122 shown in Figure 8 illustrates design parameters including head offset, neck height, neck angle, length, and stem size. The dimensional reference 122 helps the surgeon to identify design parameters while he/she is editing the template 32.

Other examples, of the surgeon's device 12 also access tools 16 for creating a design based on diagnostic data 14. As discussed above, diagnostic data 14 can include X-rays, MRI images, or CT images. In some examples, the tools 16 can enable the surgeon to select certain points or elements associated with the diagnostic data 14. The automated platform 22 can be provided with tools 26 to translate the selected points into a design. For example, there could be an X-ray showing a fractured leg, and the surgeon can select two points on one side of the fracture, and one point on the other side of the fracture. The automated platform 22 could translate those points into a design for an intramedullary nail.

In some examples a working model 118 can be provided on the surgeon's device 12 to allow the surgeon to view the progress of the design, and to catch any potential mistakes. The surgeon can enter edits, and can select a regeneration button 120 to update the working model 118. Alternatively, the working model 118 can be regenerated automatically whenever the surgeon edits the template 32. For example, in Figure 8 the standard length is 130 mm, and the edited length is 140 mm. When the regeneration button 120 is selected, the working model 118 will grow 10 mm in length. If the surgeon made a mistake while entering edits, then the mistake can be illustrated on the working model 118. For example, if the length was 130 mm, and the surgeon mistakenly entered 1300 mm, then the surgeon can catch the mistake by viewing the working model 118.

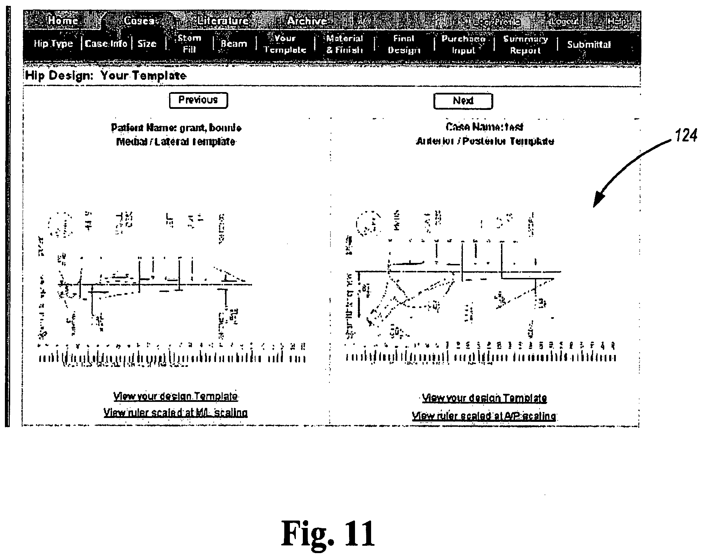

In addition, some examples of the surgeon's device 12 can include a comparison tool 124 to allow the surgeon to compare the design to diagnostic data (not shown), such as an X-ray of the patient, to the design for the implant. This comparison tool 124 is helpful because it allows the surgeon to determine whether the design is appropriate for treatment of the patient's condition. The example of the comparison tool 124 illustrated in Figure 11 is a printable illustration of the design scaled to fit the diagnostic data. Other examples of the comparison tool 124 are contemplated. In Figure 11, there is one printable illustration for the medial and lateral side of the implant, and another printable illustration for the anterior and posterior side of the implant. The surgeon can print out the illustrations and compare them to the diagnostic data. If the design on the illustrations does not fit the diagnostic data, then the surgeon can use the tools 16 for editing and redesign the implant.

Certain arrangements can include a server 20. The server 20 can be a computer, if desired. The server 20 can be enabled to receive and transmit information between at least the surgeon's device 12 and the automated platform 22. The server 20 can transmit information over a network 18, which can be the Internet, or an Intranet.

Other arrangements can include an automated platform 22. The automated platform 22 can be accessed by a biomedical implant manufacturer to practice methods of the invention. In Figure 1, the automated platform 22 is a computer, but other arrangements are contemplated. The server 20 and the automated platform 22 can be housed in the same location, or can be housed in different locations, if desired. In the example shown in Figure 1, the server 20 and the automated platform 22 are two separate elements, but in other examples, the server 20 and the automated platform 22 can be members of the same element. In some examples, memory 24 can be provided. The memory 24 can be a hard drive of a computer, if desired, but other arrangements are contemplated. The automated platform 22 can access the memory 24, but the automated platform 22 and the memory 24 need not be members of the same element. The memory 24 can store multiple types of information, including a collection of templates 42, ranges of acceptable design parameters for implants 44, or designs of standard implants 46.

Examples of the automated platform 22 can be provided with tools 26 to automatically generate certain responses to the edits 30 received from the surgeon's device 12. In some embodiments the tools 26 can include computer aided design ("CAD") systems. Common CAD systems known in the art can include SolidWorks®, produced by SolidWorks Corporation, 300 Baker Avenue, Concord, MA 01742, or Pro Engineer®, produced by Parametric Technology Corporation, 140 Kendrick Street, Needham, MA 02494. The CAD systems can be enabled to translate edits 30 of the template 32 received from the surgeon's device 12 into a design for an implant 34.

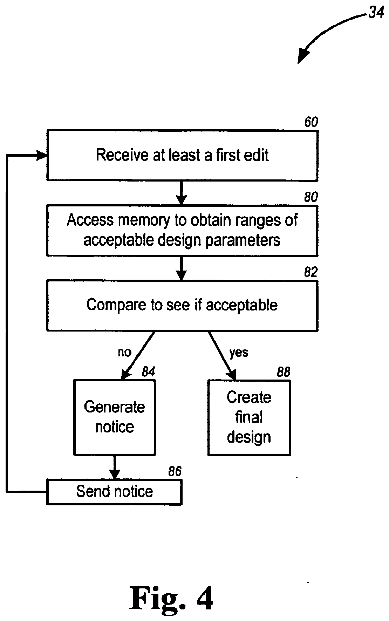

In some examples, the automated platform 22 can have tools 26 to ensure that the design is acceptable for treatment of a patient's condition. Not every type of biomedical implant is appropriate for insertion into the human body, and sometimes the surgeon might not be aware of whether a particular design is acceptable. For example, a regulatory agency, such as the Food and Drug Administration, might have determined that a hip stem cannot exceed a certain weight. The surgeon might not be aware of the ranges of acceptable weights, and might have designed an implant with an unacceptable weight. Accordingly, tools 26 can be provided to practice a method, illustrated in Figure 4, to ensure that the design is acceptable for treatment of a patient's condition. In some examples there can be ranges of acceptable design parameters 44 to identify whether an edit 30 is acceptable. In step 80 of the illustrated example, the automated platform 22 can access memory 24 to obtain the ranges of acceptable design parameters 44. Next, in step 82, the automated platform 22 can compare the edit 30 to the ranges of acceptable design parameters 44 to determine if the edit 30 is acceptable. If the edit 30 is not acceptable, then in step 84 the automated platform 22 can generate a notice, and in step 86 the automated platform 22 can send the notice to the server 20, and in turn to the surgeon's device 12. The notice informs the surgeon that the edit 30 is not acceptable, and can provide the surgeon with an opportunity to enter another edit. In some arrangements the automated platform 22 is further enabled to repeat the process as needed by comparing any successive edits to the ranges of acceptable design parameters 44. If the edit 30 is acceptable, then the automated platform 22 can create a final design for the biomedical implant, or can provide the surgeon with an opportunity to enter another edit. Accordingly, the automated platform 22 can compare an edit 30 to ranges of acceptable design parameters 44.

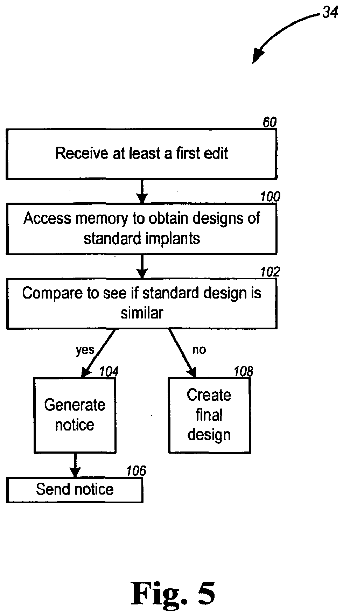

Alternatively, in some examples, the automated platform 22 can have tools 26 to compare an edit 30 to the design of a standard biomedical implant 46. Biomedical implant manufacturers have available a wide variety of standard biomedical implants. Sometimes the surgeon might not be aware that a standard implant is suitable for his/her application. The surgeon might find it desirable to obtain a standard implant because a standard implant is less expensive and more readily available than a customized implant. Accordingly, tools 26 can be provided to practice a method, illustrated in Figure 5, to notify the surgeon that a standard implant might be acceptable for the application. In step 100, the automated platform 22 can access memory 24 to obtain designs of standard implants 46. Next, in step 102, the automated platform 22 can compare the edit 30 to the designs of standard implants 46. If the edit 30 is substantially similar to a design of a standard implant 46, then in step 104 the automated platform 22 can generate a notice, and in step 106 the automated platform 22 can send the notice to the server 20, and in turn to the surgeon's device 12. The notice can inform the surgeon that a standard implant is similar to the edit 30, and can provide the surgeon with an opportunity to obtain the standard implant. If the edit 30 is not substantially similar to the design of a standard implant 46, then in step 108 of certain embodiments the automated platform 22 can create a final design, or can provide the surgeon with an opportunity to enter another. Accordingly, the automated platform can compare the edit 30 to the designs of standard implants 46.

In certain examples the automated platform 22 is further enabled to generate manufacturing instructions 36 to manufacture the implant 40. The manufacturing instructions 36 can include computer aided manufacturing ("CAM") instructions. The manufacturing instructions 36 can be sent to a manufacturing facility 38. In certain arrangements the server 20 and the network 18 can send the manufacturing instructions 36 to the manufacturing facility 38, while in other arrangements the manufacturing instructions 36 can be sent by other means, such as by mail or courier. The manufacturing facility 38 can include standard machine equipment to manufacture implants. When the biomedical implant 40 is completed, the manufacturing facility 38 can send the implant 40 to the surgeon. In some examples, the biomedical implant 40 can be sent by the mail.

Referring now to Figures 1-2, certain methods will now be discussed. In step 50 the surgeon uses the surgeon's device 12 to enter information 14, such as patient data or diagnostic data, and forms a request 28 for a template 32 that represents a biomedical implant. In some arrangements there can be memory associated with the surgeon's device 12, and the memory can be provided with multiple types biomedical implant templates 42. The surgeon's device 12 can access the memory, and can retrieve the specific template 32 that corresponds to the request 28. In other examples such as illustrated in step 52, the surgeon's device 12 transmits the information 14 and the request 28 for a template 32 to the server 20, which in turn transmits to the automated platform 22. In step 54, the automated platform 22 can then accesses memory 24. The information 14 can be stored in memory 24 to keep a record of the surgeon's order for a biomedical implant 40. In this manner, the information 14 can be recalled if the surgeon needed to temporarily pause the order, or if the surgeon needed to reopen the order once completed. In certain examples the memory 24 can store multiple types biomedical implant templates 42, and can obtain the template 32 that corresponds to the surgeon's request 28. In step 56, the memory 24 can then deliver the template 32 to the automated platform 22, which in turn can transmit the template 32 to the server 20, which in turn can transmit the template 32 to the surgeon's device 12.

In step 58, the surgeon can use tools 16 for editing that are accessible by the surgeon's device 12 to create at least a first edit 30 to the template 32. The tools 16 can be provided on the surgeon's device 12, or can be provided on the automatic platform 22 or the server 20 and can be accessible by the surgeon's device 12. As discussed above, certain arrangements can provide tools 16 for editing comprising at least one of a slideable bar 110, a menu 112, a blank field 114, a selector 116, or a combination of any of these. Whatever arrangement of the tool 16 for editing is provided, in step 58 the surgeon can use the tool 16 to generate at least a first edit 30 on the template 32. In step 60, the at least a first edit 30 can be transmitted to the server 20, which in turn can transmit the at least a first edit 30 to the automated platform 22.

In step 62, the automated platform 22 can use tools 26 to generate at least a first response 34. As discussed above, the at least a first response 34 can be to translate the at least a first edit 30 into an at least a first design (not shown). In the example illustrated in Figure 4, the at least a first response 34 can be to ensure that the design is acceptable for treatment of a patient's condition. In step 84 of Figure 4, the automated platform 22 can generate a notice if the at least a first edit 30 falls outside of the ranges of acceptable design parameters 44. Alternatively, in the example illustrated in Figure 5, the at least a first response 34 can be to compare the at least a first design 30 to the designs of standard implants 46. The automated platform 22 can generate a notice if the at least a first edit 30 is substantially similar to the design parameters of standard implants 46. In still other arrangements, the at least a first response 34 can be a combination of any of these responses. Still other responses are contemplated. In step 64, the at least a first response 34 can be transmitted to the server 20, which in turn can transmit the at least a first response 34 to the surgeon's device 12.

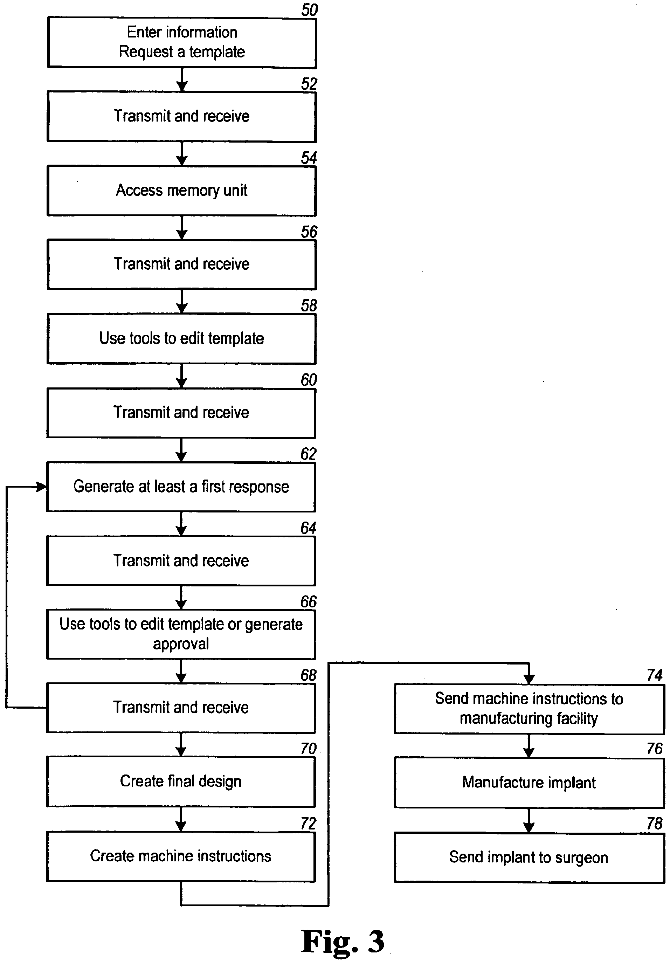

In step 66, the surgeon's device 12 can either use the tools 16 for editing to generate at least a second edit (not shown), or can generate approval of the design (not shown). If the at least a first response 34 was to generate at least a first design, then the surgeon could give approval of the design. Alternatively, the surgeon might not be satisfied with the at least a first design, and can use the tools 16 for editing to generate at least a second edit. If the at least a first response 34 was to generate notice that at least a first edit 30 falls outside of the ranges of acceptable design parameters 44, then the surgeon can use the tools 16 for editing to generate at least a second edit. Finally, if the at least a first response 34 was to generate notice that the at least a first edit 30 is substantially similar to the design of a standard implant 46, then the surgeon can give approval for the standard implant. In step 68, the approval or the second edit can be transmitted to the server 20, which in turn can transmit the approval or the second edit to the automated platform 22. As indicated in Figure 3, certain arrangements allow for repetition of steps 62 through 68 on successive edits by the surgeon's device 12.

In step 70, the automated platform 22 can create a final design (not shown). The final design is the product of the collaboration of the surgeon and the biomedical implant manufacturer. The surgeon contributed the at least a first edit 30, and the automated platform 22 of the biomedical implant manufacturer contributed at least a first response 34. In step 72, the automated platform 22 can create manufacturing instructions 36 to enable a manufacturing facility 38 to manufacture the implant, if desired. In step 74, the automated platform 22 can send the manufacturing instructions 36 to a manufacturing facility 38. In certain arrangements the server 20 can send the manufacturing instructions 36 to the manufacturing facility 38, while in other arrangements the manufacturing instructions 36 can be sent by other means, such as by mail or courier. In step 76, the manufacturing facility 38 can make the implant 40, and in step 78, the manufacturing facility 38 can send the implant 40 to the surgeon. The implant 40 can be sent to the surgeon by many means, including for example through the mail or courier.

### Figures 12 Through 13

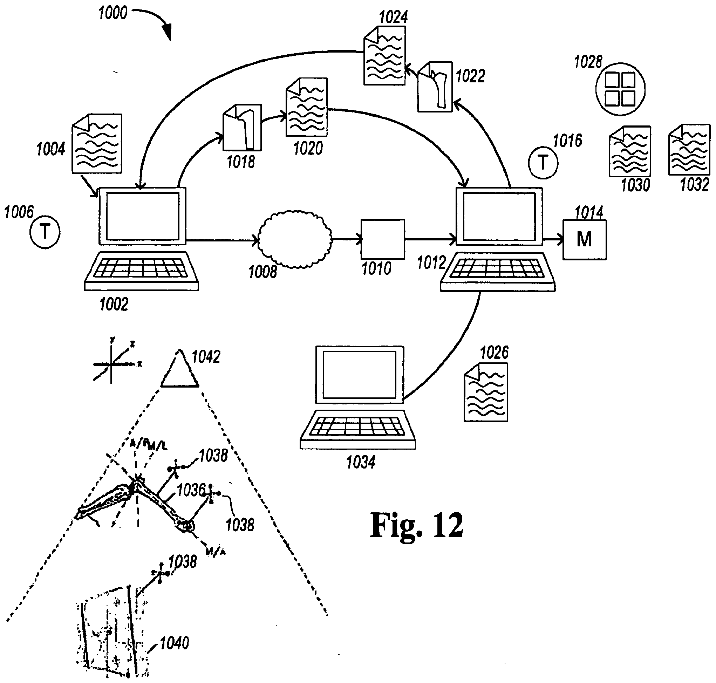

While the following exemplary arrangements of Figures 12-13 relate to procedures and implants for knee arthroplasty, at least some arrangements are not limited to any particular surgical technique or medical device type. The examples illustrate general principles that are applicable for a variety of surgical techniques and medical devices.

Certain examples involve planning and/or executing an arthroplasty surgical procedure. The arthroplasty surgical procedure plan may utilize information received from a surgeon or other person about the surgical site, including, for example, the location and/or orientation of resection cuts. The surgeon or other person may make such selections, for example, based on information about bone quality and the articular geometry of the surgical site. The surgeon and/or other persons may further participate in the selection or design of aspects of custom medical implant designed for use in the planned surgical procedure. So, for example, a surgeon may specify a custom implant that would have "box cuts" positioned differently than otherwise and be able to adjust, in the surgical plan, the target location for the cuts that allow proper placement of the component.

Accordingly, one example involves a method of planning and executing a medical implant surgical procedure that may involve receiving information about a surgical site of a patient. The method may further involve using the information about the surgical site to determine a surgical procedure step for creating an anatomic structure attribute that allows attachment of a medical implant. The method may further involve using information about the anatomic structure attribute to determine a custom attribute of the medical implant for attaching the medical implant to the anatomic structure attribute. The method may further involve creating the medical implant comprising the custom attribute and implanting the medical implant at the surgical site by (i) following the surgical procedure step and (ii) attaching the medical implant custom attribute to the anatomic structure attribute.

The method may involve estimating implant performance after surgeries given potential combinations of potential medical implant attributes and potential anatomic structure attributes and/or determinations of additional medical equipment required in surgeries involving potential combinations of the potential medical implant attributes and potential anatomic structure attributes. The method may involve estimating the difficulty of surgeries involving potential combinations of the potential medical implant attributes and potential anatomic structure attributes and/or estimations of the minimum levels of skill required of a surgeon in surgeries involving potential combinations of the potential medical implant attributes and potential anatomic structure attributes.

Certain examples provide systems for planning a medical implant surgical procedure. Such systems may involve components that allow users (from surgeons to medical device manufacturing specialists to finite element analysis experts and others) to enter information, share information, or otherwise collaborate or contribute to the planning and execution of a medical procedure and provision of an appropriate medical implant. For example, one arrangement involves a computer system comprising a user interface that (1) allows a user, such as a surgeon, to enter information about a surgical site, and (2) a display component that provides an image representative of a custom attribute of a medical implant for allowing attachment at the surgical site and an image representative of an attribute of an altered anatomic structure attribute for attachment of a the custom attribute of the medical implant. The system may allow the user to make adjustments that result in changes to the image representative of the custom attribute of the medical device and the image representative of the attribute of the altered anatomic structure. Changes made using the user interface to one image (i.e. the implant or the anatomy) may change both the image of the implant and the image of the anatomy, i.e., automatically adjusting one based on the change made to the other.

The user interface may further allow medical device manufacturing specialists and others to participate in the planning process and/or contribute by providing information about the sensitivity of the surgical procedure to changes in technique and implant dimensions, the sensitivity of the implant's performance to changes in implant dimensions and surgical procedure, and/or the feasibility of the combination with respect to level of skill of the surgeon and additional required medical equipment, (guides, cutting blocks, etc). Finite element analysis and/or other modeling or simulation techniques may also or alternatively be used to generate one or more proposed device/anatomy combinations.

Certain examples involve computer aided surgery. Data, including information regarding the surgery and custom device (e.g., custom cuts and/or custom implants), is provided to a CAS system. In addition, this information could also contain 3D model geometry for use by the CAS system to display an image representative of an actual custom implant being used. During surgery, the CAS system facilitates the placement of the "custom cuts" using standard or alternative instrumentation, typically computer controlled and not encumbered by mechanical alignment means. Thus, one major advantage is that slight computer controlled modifications to cut location and/or orientation may allow arthroplasty for patients with larger deformities that might otherwise be contraindicated. Systems and methods in accordance with these examples could thus facilitate a primary knee system.

For example, the method may involve a method of planning and executing a medical implant surgical procedure involving receiving input relating to an attribute of an anatomic structure for attachment of an implant. The method may further involve using the input to determine a custom attribute of the implant for attachment to the anatomic structure attribute. The method may further involve providing to a CAS system (i) information about the custom implant attribute and (ii) information about the anatomic structure attribute. The method may further involve providing during surgery from the CAS system (i) an image representative of the custom implant attribute and (ii) an image representative of the anatomic structure attribute. The images may overlap, be adjacent or otherwise be available for viewing or use by the surgeon. The CAS system may further provide information regarding surgical steps associated with surgically modifying the patient's anatomy to have the designed physical attributes using images or information about the attributes from a pre-surgery plan.

Referring now to Figure 12, system 1000 of certain examples can be provided with a surgeon's design device 1002. In Figure 12, the surgeon's design device 1002 is a computer, but other examples can include a facsimile machine, a telephone, a handheld computing device, or a pager. The surgeon's design device 1002 can access a tool 1006 to input information 1004. The tool 1006 can be provided on the surgeon's design device 1002, or can be provided on the automatic platform 1012 or the server 1010 and can be accessible by the surgeon's design device 1002. Information 1004 can include identifying information for the surgeon and/or the patient, including names, case numbers, and insurance information, if desired. In certain examples, information 1004 can include diagnostic data, such as a digital X-ray image, a magnetic resonance image ("MRI"), or a computer tomography ("CT") image. The surgeon's design device 1002 can access other tools 1006 that can be provided on the surgeon's design device 1002, or can be provided on the automatic platform 1012 or the server 1010 and can be accessible by the surgeon's design device 1002. One tool 1006 can enable the surgeon to send a request 1018 for a template 1022 that represents a biomedical implant, an accessory to a biomedical implant, and/or represents one or more exemplary anatomic structures, features, or attributes. Such a tool 1006 may provide the surgeon with various choices, such as a choice of implant type.

Certain examples also provide tools 1006 for editing at least one design parameter or attribute of a surgical implant template 1022. The tools 1006 can be provided on the surgeon's design device 1002, or can be provided on the automatic platform 1012 or the server 1010 and can be accessible by the surgeon's design device 1002. The tool 1006 can further include one or more forms to input one or more design parameters.

Certain examples provide tools 1006 for editing at least one potential design parameter or attribute of a surgical site altered for attachment of a surgical implant. As with other tools, such a surgical site design tool can be provided on the automatic platform 1012 or the server 1010 and can be accessible by the surgeon's design device 1002 and can include one or more forms to input one or more design parameters. For example, a form embodied as a menu may allow a surgeon to click on the down arrow to obtain a list of design parameters and attributes, and then click to select the desired design parameter of his/her choice. A dimensional reference can serve as a guide while the surgeon is editing a template. Such a reference can help a surgeon to identify design parameters and attributes while he/she is editing a template.

Other examples of the surgeon's design device 1002 also access tools 1006 for creating an implant or altered surgical site design based on diagnostic data 1004. As discussed above, diagnostic data 1004 can include, among other things, X-rays, MRI images, or CT images. In some arrangements, the tools 1006 can enable the surgeon to select certain points or elements associated with the diagnostic data 1004. The automated platform 1012 can be provided with tools 1016 to translate the selected points into a design. In some arrangements, a working model of an implant and/or an altered surgical site can be provided on the surgeon's design device 1002 to allow the surgeon to view progress, catch potential mistakes, and edit the design.

Certain arrangements can include a server 1010. The server 1010 can be a computer, if desired. The server 1010 can be enabled to receive and transmit information between at least the surgeon's design device 1002 and the automated platform 1012. The server 1010 can transmit information over a network 1008, which can be the Internet, or an Intranet. Other arrangements can include an automated platform 1012. The automated platform 1012 can be accessed by a biomedical implant manufacturer to practice methods of the invention. In Figure 12, the automated platform 1012 is a computer, but other arrangements are contemplated. The server 1010 and the automated platform 1012 can be housed in the same location, or can be housed in different locations, if desired. In the arrangement shown in Figure 12, the server 1010 and the automated platform 1012 are two separate elements, but in other arrangements, the server 1010 and the automated platform 1012 can be members of the same element. In some arrangements, memory 1014 can be provided. The memory 1014 can be a hard drive of a computer, if desired, but other arrangements are contemplated. The automated platform 1012 can access the memory 1014, but the automated platform 1012 and the memory 1014 need not be members of the same element. The memory 1014 can store multiple types of information, including a collection of templates 1028, ranges of acceptable design parameters and attributes for implants and associated anatomic structures 1030, and/or designs of standard implants and associated anatomic structures for attachment 1032.

The automated platform 1012 can be provided with tools 1016 to automatically generate certain responses to the edits 1020 received from the surgeon's design device 1002. In some arrangements, the tools 1016 can include computer aided design ("CAD") systems. Common CAD systems known in the art can include SolidWorks®, produced by SolidWorks Corporation, 300 Baker Avenue, Concord, MA 01742, or Pro Engineer®, produced by Parametric Technology Corporation, 140 Kendrick Street, Needham, MA 02494. The CAD systems can be enabled to translate edits 1020 of the template 1022 received from the surgeon's design device 1002 into a design for an implant 1024 or altered surgical site.

In some examples the automated platform 1012 can have tools 1016 to ensure that the design is acceptable for treatment of a patient's condition. Not every type of biomedical implant is appropriate for insertion into the human body, and sometimes the surgeon might not be aware of whether a particular design is acceptable. For example, a regulatory agency, such as the Food and Drug Administration, might have determined that a hip stem cannot exceed a certain weight. The surgeon might not be aware of the ranges of acceptable weights, and might have designed an implant with an unacceptable weight. Similarly, certain injuries or anatomical conditions may make the use of certain implants unacceptable. Accordingly, tools 1016 can be provided to ensure that the design of an implant and/or associated altered anatomic attributes are acceptable for treatment of a patient's condition.

In some examples there can be ranges of acceptable design parameters 1030 to identify whether an edit 1020 is acceptable. This informs the surgeon that the edit 1020 is not acceptable, and can provide the surgeon with an opportunity to enter another edit. In some examples, the automated platform 1012 is further enabled to repeat the process as needed by comparing any successive edits to the ranges of acceptable design parameters 1030. If the edit 1020 is acceptable, then in some examples the automated platform 1012 can create a final design for the biomedical implant, or can provide the surgeon with an opportunity to enter another edit. Accordingly, in some arrangements, the automated platform 1012 can compare an edit 1020 to ranges of acceptable design parameters 1030.

Alternatively, the automated platform 1012 can have tools 1016 to compare a surgeon's design to the standard designs 1032. Biomedical implant manufacturers have available a wide variety of standard biomedical implants that attach to a variety of anatomic structures. The surgeon might not be aware that a standard implant is suitable for his/her application. The surgeon might find it desirable to obtain a standard implant because a standard implant is less expensive and more readily available. Accordingly, tools 1016 can be provided to practice a method to notify the surgeon that a standard implant might be acceptable for the application. In certain arrangements, the automated platform 1012 is further enabled to generate manufacturing instructions (e.g., CAM instructions) that may be automatically sent to a manufacturing facility.

Figure 12 further illustrates computer assisted surgery components 1034, 1036, 1038, 1040, 1042 that can receive surgical implant and surgical procedure information 1026 from the other system components, e.g., from the automated platform 1012, for use during a surgical procedure. This information 1026 can include information about attributes of a proposed implant 1040 as well as information about attributes of the proposed altered anatomy 1036 to which the implant 1040 is intended to be attached. In certain examples, the computer assisted surgery system 1034 allows preoperative planning that utilizes this information 1026 in which the surgeon selects reference points and determines implant position. The preoperative CAS planning may occur in conjunction with the design of the implant 1040 and/or the design of the altered anatomic structures 1036 for attachment of the implant 1040.

Certain arrangements involve computer assisted surgery for tracking anatomy 1036, implants 1040, instrumentation (not shown), virtual constructs (not shown), rendering images shown, for example, on a monitor of system 1034, and data (not shown) related to them in connection with the surgical operation in which anatomic structures 1036 are altered (e.g., by resection) and implants 1040 are attached to the altered anatomic structures 1036. Anatomical structures 1036 and various items (e.g., implants 1040) may be attached to or otherwise associated with fiducial functionality 1038, and constructs (not shown) may be registered in position using fiducial functionality 1038 whose position and orientation can be sensed and tracked. Such structures 1036, items 1040 and constructs can be rendered onscreen properly positioned and oriented relative to each other using associated image files, data files, image input, other sensory input, and/or based on the tracking. This allows surgeons to navigate and perform surgeries using images that reveal interior portions of the body combined with computer generated or transmitted images that show surgical implants 1040 and/or other devices located, oriented, and/or attached properly to the body parts 1036.

The use of a CAS system may allow more accurate and effective resection of bone, placement and assessment of implants and joint performance, and placement and assessment of performance of actual implants and joint performance. Various alignment modules and other structures and processes may allow for coarse and fine alignment of instrumentation and other devices relative to bone for use in connection with the tracking systems, as explained in U.S. Patent Publication 2003/0069591 entitled "COMPUTER ASSISTED KNEE ARTHROPLASTY INSTRUMENTATION, SYSTEMS, AND PROCESSES".

Intraoperatively, the CAS system 1034 may make use of CT scans, MRI data, digitized points on the anatomy, and other images and information and may calibrate patient position to the preoperative plan, such as using a "point cloud" technique, and can use a robot to make bone preparations. Position and/or orientation tracking sensors, such as infrared sensors, acting stereoscopically or otherwise, may be used to track positions of body parts 1036, surgery-related items 1040, and virtual constructs or references such as rotational axes which have been calculated and stored based on designation of bone landmarks. Processing capability, such as any desired form of computer functionality, whether standalone, networked, or otherwise, may take into account the position and orientation information as to various items in the position sensing field (which may correspond generally or specifically to all or portions or more than all of the surgical field) based on sensed position and orientation of their associated fiducials 1038 or based on stored position and/or orientation information. The processing functionality correlates this position and orientation information for each object with stored information regarding the items, such as a computerized fluoroscopic imaged file of a femur or tibia, a wire frame data file for rendering a representation of an instrumentation component, trial prosthesis or actual prosthesis, or a computer generated file relating to a rotational axis or other virtual construct or reference. The processing functionality then displays position and orientation of these objects on a screen or monitor, or otherwise. Thus, it can display and otherwise output useful data relating to predicted or actual position, orientation, and altered structural attributes of body parts 1036, implants 1040, and other items and virtual constructs for use in navigation, assessment, and otherwise performing surgery or other operations.

As one example, images such as fluoroscopy images showing internal aspects of the femur and tibia can be displayed on a monitor in combination with actual or predicted shape, position and orientation of surgical implants and altered anatomic structure in order to allow the surgeon to properly position and assess performance of various aspects of the joint being repaired, reconstructed or replaced. The surgeon may use this preoperatively to design an appropriate surgical implant 1040 and corresponding altered anatomic structure attributes. The surgeon may then interoperatively use navigation tools, instrumentation, trial prostheses, actual prostheses and other items relative to bones and other body parts in order to perform surgeries more accurately, efficiently, and with better alignment and stability. This system may also generate data based on position tracking and, if desired, other information to provide cues on screen, aurally or as otherwise desired to assist in the surgery such as suggesting certain surgical steps in accordance with a predefined surgical plan, e.g., bone modification steps. Moreover, interoperatively such bone modification steps may be modified based on actual surgical conditions, e.g., automatically requiring or suggesting that a surgeon release certain ligaments or portions of them based on the actual performance of components as sensed by the instrumentation, systems, and processes of the CAS system.

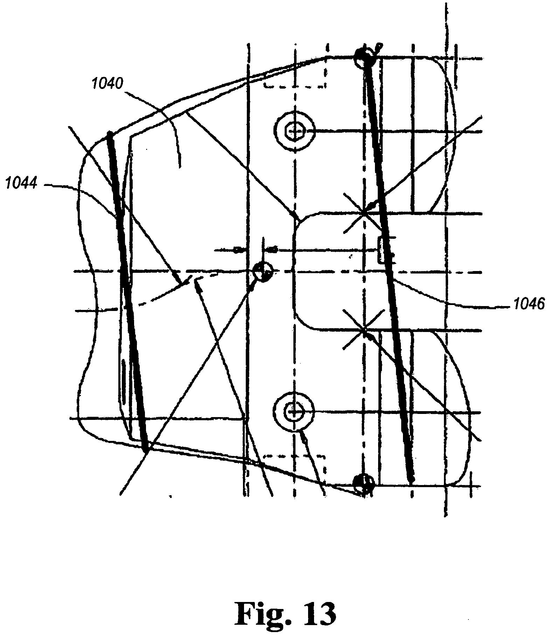

Referring now to Figure 13 of an exemplary implant illustrating non-standard geometry according to certain embodiments of the invention. Specifically, a femoral knee implant 1040 is illustrated having attributes 1044, 1046 presenting non-standard geometries for attachment to a femur bone with appropriate resections. This custom implant 1040 is therefore capable of attaching to bones that cannot or should not be resected in standard resection locations, expanding the medical circumstances in which a surgical implant may be used.

### Figures 14 Through 18

Some other arrangements may provide a computer or website that allows a user to load images of a fractured bone and specify a particular configuration of fixation and compression screws with a plate on the loaded bone. The computer is able to replicate normal stresses imposed on the bone by regular activities using finite element analysis. The loaded images may originate from radiographs of the actual fractured bone or may be a predefined bone structure already stored. In one arrangement, the computer is also able to predict which combination of screws will work best. This approach can be applied to any bone/device construct including nails, external fixators, and other products. Also, this system can be developed as a web based system using advanced software analysis tools or as a stand alone system that is programmed for the specific application.

In certain arrangements, the system allows a surgeon or other person to access a website and input information about a patient's fracture and the plate, or other device being considered. The surgeon or other user may also input information about the particular patient, such as age, weight, height, etc., and/or information about the expected loads or stresses to which the bone may be subjected. Using the inputted information elements, the system performs one or more of a variety of mechanical and biomechanical analysis and returns information to the surgeon or other user that may be used to optimize or otherwise enhance patient treatment.

The analysis may utilize specific information about the patient or may use normal, average, or other appropriate data when patient specific data is not provided or otherwise cannot be used. This approach can be applied to any bone/device construct including nails, external fixators, and other products. Also, this system can be developed as a web based system using advanced software analysis tools or as a stand alone system that is programmed for the specific application. The system may utilize prior laboratory results regarding the performance of possible bone/device constructs. For example, in the case of bone plates, by performing laboratory biomechanical studies, various fracture/plate constructs can be evaluated. Based on this information, a computer simulation model can be developed that correlates and defines all the possible permutations of fracture type and plate construct. Such simulations can provide surgeons and other users valuable information about potential failure points and stress distribution for various medical device configurations being considered. In addition to simulations, systems according to the present invention can provide reference information, such as database information about common fracture types and bone conditions and statistics about medical device configurations used to treat fracture types and bone conditions. Such information can also aid the surgeon or other user in selecting an appropriate means for treating a patient's bone condition.

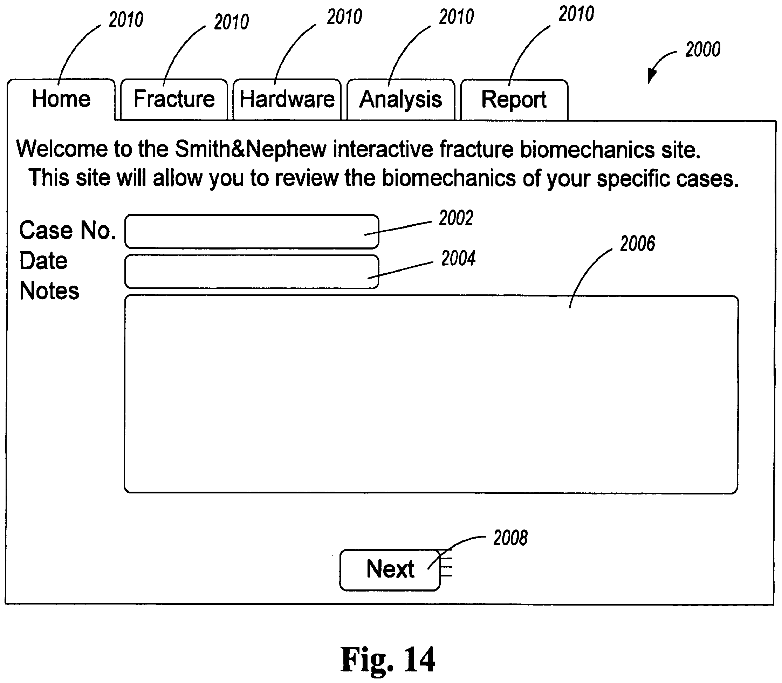

Figures 14-18 illustrate an exemplary computer interface for a simulation system. The computer simulation may utilize a finite element analysis of the bone/plate construct that shows the surgeon how to optimize the construct for the appropriate stiffness and stress. A standard computer may be used and will typically include processing functionality, memory functionality, input functionality and output functionality. The system may utilize a variety of software and network components to provide a user interface, remote access, shared access, and to perform and report any analysis.

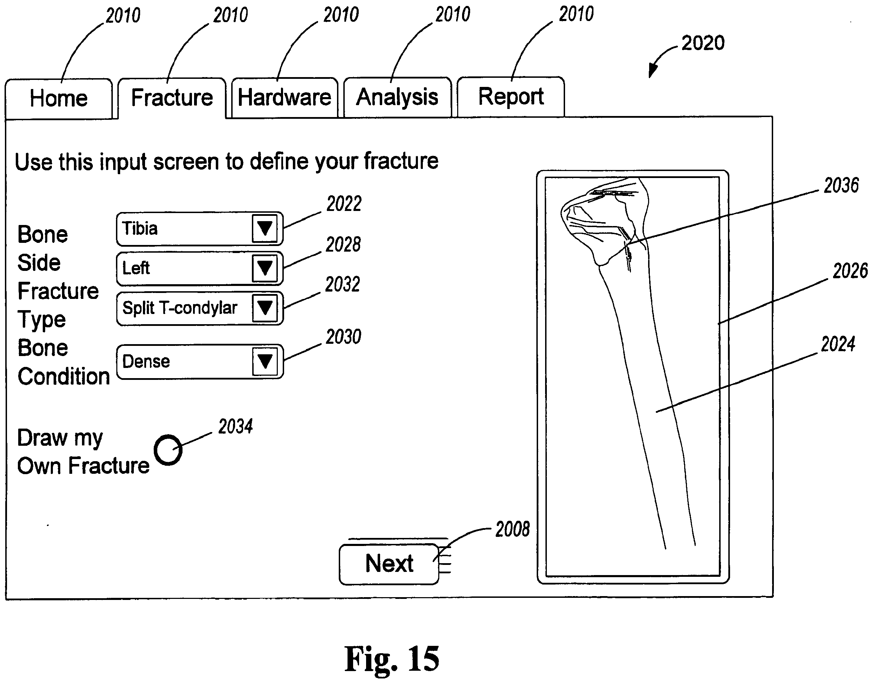

In Figures 14-18, a software program provides the exemplary computer interface for a simulation system. Figure 14 illustrates a computer user interface 2000 for inputting and displaying information about a patient case. The user interface 2000 allows a surgeon or other user to enter a case number 2002, a date 2004, and notes 2006. Other information may be entered about the case, such as planned surgery date, surgeon name, surgeon contact information, as well as any other suitable or appropriate information. A next button 2008 allows a surgeon or other user to proceed or continue to further interface screens such as the screen of Figure 15. A series of tabs 2010 also allows navigation to other portions of the user interface associated with the case.

Figure 15 illustrates a computer interface 2020 for inputting and displaying information about a bone and/or fracture. In certain arrangements, the computer interface will display an image of a bone. For example, as illustrated in Figure 15, the interface may allow a surgeon or other user to input a bone (e.g., femur, tibia, fibula, calcaneous, humerus, etc.), in a bone field 2022. Upon identification of a bone, the computer interface 2020, may display an image of that bone, or a portion of that bone. For example, in Figure 15, upon selection of "tibia," a tibia 2024 may be displayed in a window 2026 of the interface 2020. The interface 2020 may also allow a surgeon or other user to specify a side 2028. Such selection may also be reflected in the image of the bone 2024 (i.e., the image may show the left side of the bone, or the proximal end of the bone, etc.).

The interface 2020 may also allow a surgeon to input an image of a bone from another source. For example, the user may identify a photograph, scan, x-ray, digital image, or other type of image of the patient's own bone. Alternatively, the mechanical axis and/or bone landmarks may be inputted by the user (or identified using pattern recognition functionality or the like) to be used as information elements in the simulation. The software may recognize and model the bone based on the scanned image to create data for the simulation. The interface may also offer pictures or other images of bones from which the surgeon or other user may select. For example, after a user has identified a "tibia," the interface may provide images of various tibias that have been laboratory tested. Thus, the system can give users several combinations for various "standard" configurations of fractures for the surgeon to use as a benchmark at least. Upon selection of a "tibia" image that most closely or appropriately resembles or represents the patient's tibia, the software may update the image of the tibia 2024 on the interface 2020 and use one or more information elements regarding the specific tibia inputted or otherwise selected in a simulation or modeling that is ultimately executed.

In Figure 15, the interface allows a surgeon or other user to input a bone condition 2030. In certain arrangements, the surgeon or other user may input additional information about the bone that allows the software to more accurately model the bone. For example, the surgeon or other user may specify specific bone dimension, perhaps measured on an x-ray. Entry of additional information about the bone may allow the software to more accurately display and model the bone/fracture/device combination that is being evaluated.

In the interface 2020 shown in Figure 15, the surgeon or other user also has the ability to select a fracture type 2032 or "Draw my Own Fracture" 2034. Upon selection of a fracture type 2032, the interface 2020 will display a fracture 2036 of that type ("Split T-condylar") on the image of the bone 2024, displayed in window 2026. The surgeon or other user may manually move or, instead, draw the fracture by selecting "Draw my Own Fracture" 2034. For example, the user may use a computer mouse to draw the fracture by clicking the mouse button and moving the mouse-controlled curser over the location of the fracture line in the bone image 2024. The software may use this drawn-in representation of a fracture to associate or otherwise determine an associated fracture type from a plurality of known fracture types. Alternatively, the software may use the drawn-in fracture to predict a three dimensional fracture through the bone, and use this custom-defined fracture in simulations and displays. Such prediction can utilize any suitable technique, including those that account for bone density, bone type, and other bone characteristics to predict the internal location of a fracture from a drawn-in fracture on a two-dimensional image of the fracture. As yet another alternative, the software may require the surgeon to draw in a fracture on the bone from a variety of perspectives (e.g., ML, AP, etc.) such that these indications can be combined to determine an estimated path of the fracture through the interior of the bone. The software may present the user with a three dimensional image of a fracture and ask the user to confirm that the three dimensional image of the fracture adequately represents the patient's actual fracture. In still another arrangement, two or three dimensional images of the fracture may be inputted into the computer.

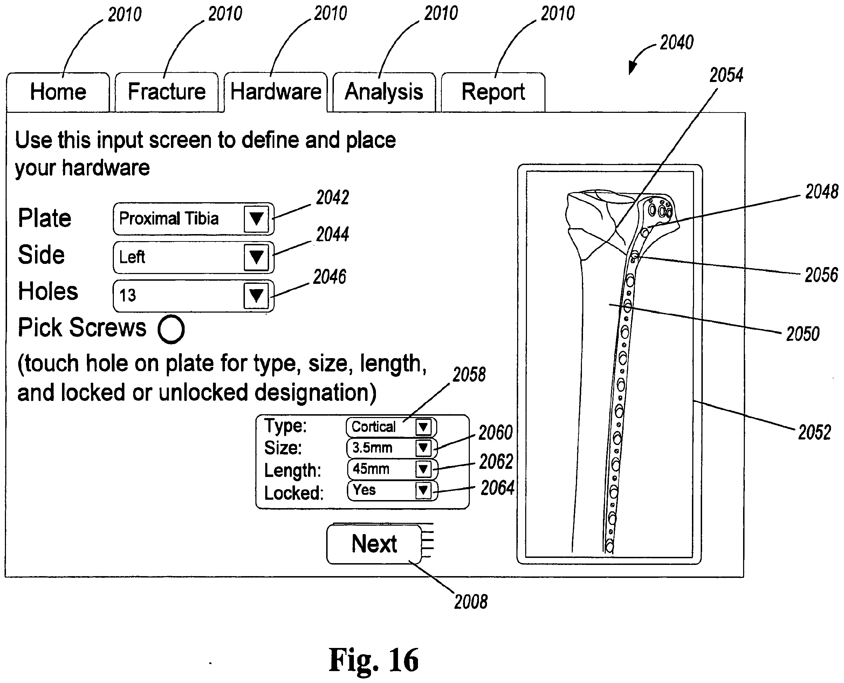

Figure 16 illustrates a computer interface 2040 for inputting and displaying information about an device, bone, and/or fracture. In this exemplary arrangement, the surgeon or other user may input a plate type 2042 (e.g., "Proximal Tibia"), a side 2044 (e.g., "Left"), and the number of screw holes in a device 2046, among other things. In certain arrangements, the plate, implant, fixator, nail or other medical device is specified allowing a surgeon to evaluate and compare different categories of medical devices (e.g., internal implants, external fixators, bone plates, and intramedullary nails).

In Figure 16, upon selection of a plate type, a plate of that type 2048 is displayed on the image of the bone 2050 in the interface image window 2052. The fracture 2054 may also be displayed. The surgeon or user may have the option to move the position of the plate 2048 relative to the bone 2050. For example, a user could use the computer mouse by moving the mouse-controlled curser over the plate 2048, clicking the mouse button, moving the mouse to move the plate 2048, and releasing the mouse once the plate 2048 appears in a desired location with respect to the bone 2050 displayed on the interface image window 2052.

The interface 2040 provides users with the option of choosing their own screws by touching or otherwise graphically pointing to one or more of the holes on the plate image 2048. For example, a user may move the mouse-controlled curser over the location of a hole 2056 in plate 2048 and click the mouse button to select a particular hole 2056 in the plate 2048. Upon this selection, the interface 2040 may prompt the user with additional input options, such as the type 2058 (e.g., "Cortical"), size 2060 (e.g., "3.5 mm"), length 2062 (e.g. "45 mm"), and whether or not the instrument is locked or not 2064 (e.g. "Yes"). The user may thus select whether or not a hole will have an instrument and thereby select a plate configuration in which one or more of the holes will use either a fixation screw, a compression screw, or some other instrument.

Accordingly, in certain arrangements, a surgeon or other user may select a medical device (e.g., plate, nail, etc.), select a configuration of instruments for use in one or more holes of the device (e.g., which holes will have screws), and select the characteristics of such instruments (e.g., size, length, type, etc.), to establish a potential construct for treatment of a patient.

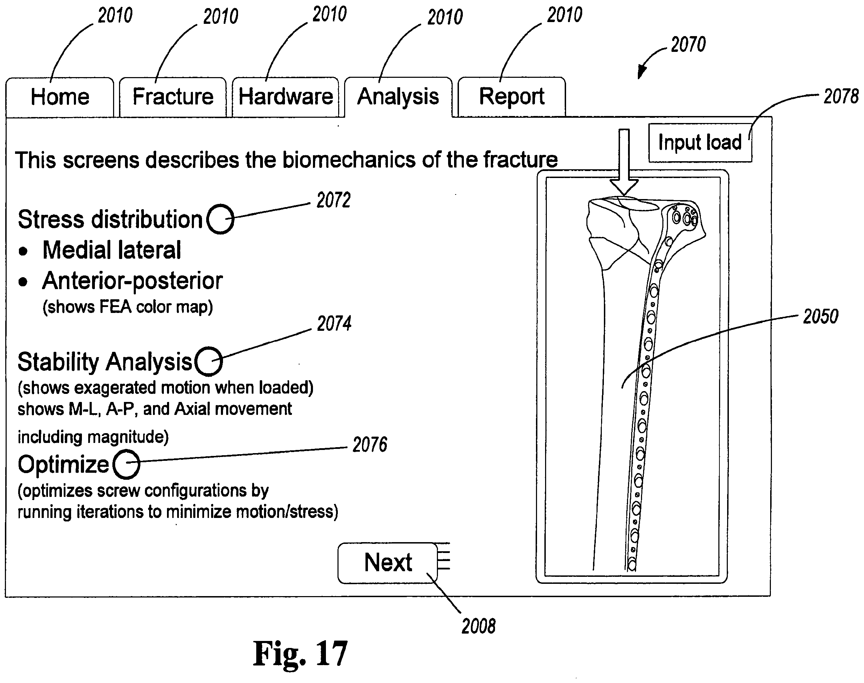

Figure 17 illustrates a computer interface 2070 for selecting analysis of stress distribution 2072, stability 2074, and/or optimization 2076. This interface 2070 allows a user to specify one or more analysis outputs 2072, 2074, 2076. The stress distribution output selection 2072 provides an analysis that simulates and illustrates the stress distribution for the particular bone/instrument construct when a given input load 2078 is applied. Such stresses can be imposed statically and/or dynamically, including over repeated cycles to predict failure of the plate and screw combination and/or bone. It may show a finite element analysis color map. The stability analysis output selection 2074 provides similar information but for exaggerated motion when loaded. It may show ML, AP, and axial movement including magnitude. The optimize selection option 2076 optimizes screw configuration by running iterations to minimize motion and/or stress. Accordingly, the outputs of certain examples include finite element analysis color maps showing stress distributions using scale of stresses, motion analysis showing exaggerated movement of construct, flags of high stress or high motion areas, and recommended changes in screw configurations to optimize fracture biomechanics. Stability stiffness of fracture/plate construct in both bending planes, torsion, and axial directions may also be provided. In addition, the output may show where high stress regions are located on the plate and thus indicate trouble spots or possible failure modes. In certain examples a computer interface will also allow a surgeon or other user to input stress conditions to further customize the results of the simulation.

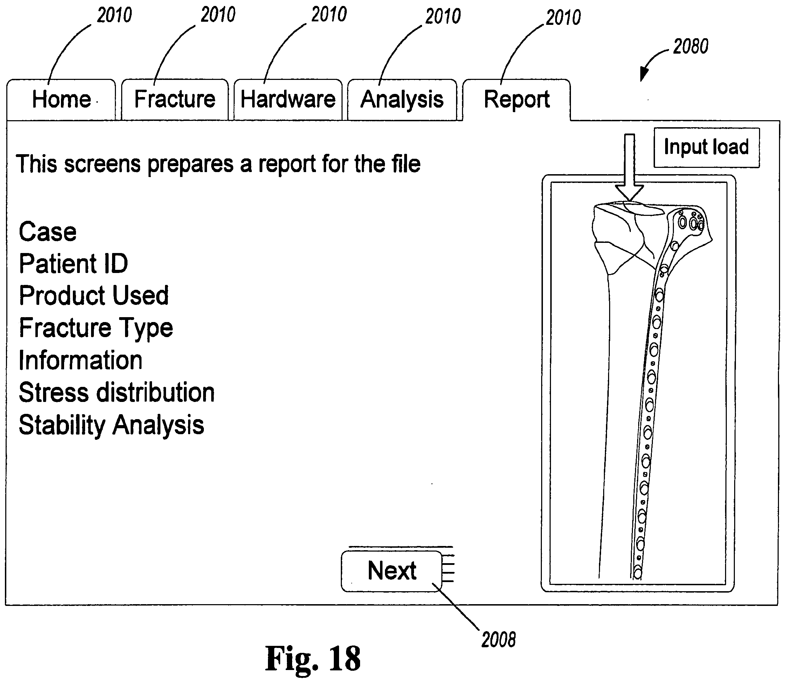

Figure 18 illustrates a computer interface 2080 for displaying a report that summarizes information inputted and displayed in the interfaces of Figure 14-17. The results of any analysis selected may also be displayed through this interface. The tabs 2010 further allow a user to go back and change inputs in any of the interfaces of Figures 14-17 and re-execute the analysis for alternative bone/fracture/instrument constructs.

The interface and software of the present invention may include programming tools as well as Computer Aided Design and analysis software, finite element modeling software, and motion analysis software. In certain examples, bone and plate models may be preloaded into the system so that as the surgeon defines the construct, the models would be pulled from a database and shown by a viewer. The software tools would take the inputs listed and produce output that would help the surgeon configure the device to optimize the biomechanics of the fracture.

The foregoing discloses certain embodiments of the present invention, and numerous modifications or alterations may be made without departing from the scope of the invention. For example, at least some embodiments of this invention may be applied to any bone/device construct including nails, external fixators, and other products. Also, some embodiments may be developed as a web based system using advanced software analysis tools or as a stand alone system that is programmed for the specific application.

## Claims

1. A method of creating an orthopaedic device, comprising:
a) receiving information about a surgical site of a patient;
b) using the information about the surgical site to determine a surgical procedure step for creating an anatomic structure attribute;
c) using information about the anatomic structure attribute to determine a custom attribute for an orthopaedic device; and
d) manufacturing the orthopaedic device to include the custom attribute; **characterised in that** using the information about the surgical site further comprises estimating a level of difficulty of the surgical procedure step.

2. The method of claim 1, further comprising estimating a minimum level of skill required to perform the surgical procedure step.

3. A method as in claim 1 or 2, wherein manufacturing the orthopaedic device comprises manufacturing a cutting block for guiding the resection of a portion of a bony anatomy associated with the surgical site.

4. A method as in claim 1 or 2, wherein manufacturing the orthopaedic device comprises manufacturing a knee implant for replacing a portion of a bony anatomy associated with the surgical site.

5. A system for creating an orthopaedic device, comprising:
a) a platform configured for
(i) receiving information about a surgical site of a patient;
(ii) determining a surgical procedure step for creating an anatomic structure attribute using the information about the surgical site; and
(iii) determining a custom attribute for an orthopaedic device using the anatomic structure attribute; and
b) at least one manufacturing device for manufacturing the orthopaedic device to include the custom attribute, **characterised in that** the platform is further configured for (iv) estimating a level of difficulty of the surgical procedure step using the information about the surgical site.

6. A system as in claim 5, wherein the platform estimates a minimum level of skill required to perform the surgical procedure step.

7. A system as in claim 5 or 6, wherein the orthopaedic device comprises a cutting block for guiding the resection of a portion of a bony anatomy associated with the surgical site.

8. A system as in claim 5 or 6, wherein the orthopaedic device comprises a knee implant for replacing a portion of a bony anatomy associated with the surgical site.

## Patentansprüche

1. Ein Verfahren zum Kreieren einer orthopädischen Vorrichtung, das Folgendes beinhaltet:
a) Erhalten von Informationen über eine Operationsstelle eines Patienten;
b) Verwenden der Informationen über die Operationsstelle zum Bestimmen eines Operationsverfahrensschritts zum Kreieren eines Attributs einer anatomischen Struktur;
c) Verwenden von Informationen über das Attribut der anatomischen Struktur zum Bestimmen eines speziellen Attributs für eine orthopädische Vorrichtung; und
d) Herstellen der das spezielle Attribut umfassenden orthopädischen Vorrichtung;
**dadurch gekennzeichnet, dass**
das Verwenden der Informationen über die Operationsstelle ferner das Schätzen eines Schwierigkeitsgrads des Operationsverfahrensschritts beinhaltet.

2. Verfahren gemäß Anspruch 1, ferner beinhaltend das Schätzen eines Mindestkompetenzgrads, der zum Durchführen des Operationsverfahrensschritts erforderlich ist.

3. Verfahren gemäß Anspruch 1 oder 2, wobei das Herstellen der orthopädischen Vorrichtung das Herstellen eines Schneideblocks zum Führen der Resektion eines Teils einer mit der Operationsstelle assoziierten Knochenanatomie beinhaltet.

4. Verfahren gemäß Anspruch 1 oder 2, wobei das Herstellen der orthopädischen Vorrichtung das Herstellen eines Knieimplantats zum Ersetzen eines Teils einer mit der Operationsstelle assoziierten Knochenanatomie beinhaltet.

5. Ein System zum Kreieren einer orthopädischen Vorrichtung, das Folgendes beinhaltet:
a) eine Plattform, die für Folgendes konfiguriert ist:
(i) Erhalten von Informationen über eine Operationsstelle eines Patienten;
(ii) Bestimmen eines Operationsverfahrensschritts zum Kreieren eines Attributs einer anatomischen Struktur unter Verwendung der Informationen über die Operationsstelle; und
(iii) Bestimmen eines speziellen Attributs für eine orthopädische Vorrichtung unter Verwendung des Attributs der anatomischen Struktur;
und
b) mindestens eine Herstellungsvorrichtung zum Herstellen der das spezielle Attribut umfassenden orthopädischen Vorrichtung, **dadurch gekennzeichnet, dass** die Plattform ferner für Folgendes konfiguriert ist: (iv) Schätzen eines Schwierigkeitsgrads des Operationsverfahrensschritts unter Verwendung der Informationen über die Operationsstelle.

6. System gemäß Anspruch 5, wobei die Plattform einen Mindestkompetenzgrad schätzt, der zum Durchführen des Operationsverfahrensschritts erforderlich ist.

7. System gemäß Anspruch 5 oder 6, wobei die orthopädische Vorrichtung einen Schneideblock zum Führen der Resektion eines Teils einer mit der Operationsstelle assoziierten Knochenanatomie beinhaltet.

8. System gemäß Anspruch 5 oder 6, wobei die orthopädische Vorrichtung ein Knieimplantat zum Ersetzen eines Teils einer mit der Operationsstelle assoziierten Knochenanatomie beinhaltet.

## Revendications

1. Une méthode de création d'un dispositif orthopédique, comprenant :
a) la réception d'informations au sujet d'un site chirurgical d'un patient ;
b) l'utilisation des informations au sujet du site chirurgical afin de déterminer une étape de procédure chirurgicale pour créer un attribut de structure anatomique ;
c) l'utilisation d'informations au sujet de l'attribut de structure anatomique afin de déterminer un attribut personnalisé pour un dispositif orthopédique ; et
d) la fabrication du dispositif orthopédique de façon à inclure l'attribut personnalisé ;
**caractérisée en ce que**
l'utilisation des informations au sujet du site chirurgical comprend en outre l'estimation d'un niveau de difficulté de l'étape de procédure chirurgicale.

2. La méthode de la revendication 1, comprenant en outre l'estimation d'un niveau de compétence minimal requis afin d'effectuer l'étape de procédure chirurgicale.

3. Une méthode telle que dans la revendication 1 ou la revendication 2, où la fabrication du dispositif orthopédique comprend la fabrication d'un bloc de coupe pour guider la résection d'une portion d'une anatomie osseuse associée au site chirurgical.

4. Une méthode telle que dans la revendication 1 ou la revendication 2, où la fabrication du dispositif orthopédique comprend la fabrication d'un implant de genou pour remplacer une portion d'une anatomie osseuse associée au site chirurgical.

5. Un système pour créer un dispositif orthopédique, comprenant :
a) une plate-forme configurée pour
(i) recevoir des informations au sujet d'un site chirurgical d'un patient ;
(ii) déterminer une étape de procédure chirurgicale pour créer un attribut de structure anatomique en utilisant les informations au sujet du site chirurgical ; et
(iii) déterminer un attribut personnalisé pour un dispositif orthopédique en utilisant l'attribut de structure anatomique ;
et
b) au moins un dispositif de fabrication pour fabriquer le dispositif orthopédique de façon à inclure l'attribut personnalisé, **caractérisé en ce que** la plate-forme est en outre configurée pour (iv) estimer un niveau de difficulté de l'étape de procédure chirurgicale en utilisant les informations au sujet du site chirurgical.

6. Un système tel que dans la revendication 5, où la plate-forme estime un niveau de compétence minimal requis afin d'effectuer l'étape de procédure chirurgicale.

7. Un système tel que dans la revendication 5 ou la revendication 6, où le dispositif orthopédique comprend un bloc de coupe pour guider la résection d'une portion d'une anatomie osseuse associée au site chirurgical.

8. Un système tel que dans la revendication 5 ou la revendication 6, où le dispositif orthopédique comprend un implant de genou pour remplacer une portion d'une anatomie osseuse associée au site chirurgical.
